(19) Europäisches Patentamt
European Patent Office
Office européen des brevets

(11) Veröffentlichungsnummer: **0 290 915 B1**

# (12) EUROPÄISCHE PATENTSCHRIFT

(45) Veröffentlichungstag der Patentschrift: **14.07.93**

(51) Int. Cl.[5]: **C07D 311/30**, C07D 405/12, C07D 413/12, A61K 31/35

(21) Anmeldenummer: **88107038.7**

(22) Anmeldetag: **03.05.88**

(54) **Flavon-3-carbonsäure-Verbindungen sowie Verfahren, Zwischenprodukte zu ihrer Herstellung und diese Verbindungen enthaltende Arzneimittel.**

(30) Priorität: **12.05.87 DE 3715779**

(43) Veröffentlichungstag der Anmeldung: **17.11.88 Patentblatt 88/46**

(45) Bekanntmachung des Hinweises auf die Patenterteilung: **14.07.93 Patentblatt 93/28**

(84) Benannte Vertragsstaaten: **BE CH DE FR GB IT LI NL**

(56) Entgegenhaltungen:
**DE-B- 1 214 696**

**CHEMICAL ABSTRACTS, Band 81, Nr. 21, 25. November 1974, Columbus, OH (US); Seite 407, Nr. 135955h&NUM;**

**JOURNAL OF THE CHEMICAL SOCIETY PERKIN TRANS. I, Nr. 4, April 1985, London (GB); A.M.B.S.R.C.S. COSTA et al., Seiten 799-808&NUM;**

(73) Patentinhaber: **Kali-Chemie Pharma GmbH**
**Hans-Böckler-Allee 20 Postfach 220**
**W-3000 Hannover 1(DE)**

(72) Erfinder: **Walenta, Rainer**
**Jürsegrund 12**
**W-3002 Wedemark 1(DE)**
Erfinder: **Müller-Peddinghaus, Reiner**
**Römerfeld 14**
**W-5060 Bergisch-Gladbach 2(DE)**
Erfinder: **Ban, Ivan**
**Hedwigsweg 10**
**W-3000 Hannover 72(DE)**
Erfinder: **Wurl, Michael**
**Brosangstrasse 7**
**W-3008 Garbsen 5(DE)**
Erfinder: **Preuschoff, Ulf**
**Sonnenweg 8**
**W-3014 Laatzen(DE)**

(74) Vertreter: **Lauer, Dieter, Dr.**
**c/o Kali-Chemie Aktiengesellschaft Postfach 220**
**W-3000 Hannover 1 (DE)**

EP 0 290 915 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue basische Flavon-3-carbonsäureamid- und -ester-Verbindungen und deren Salze und N-Oxide sowie diese Verbindungen enthaltende pharmazeutische Zubereitungen und Verfahren zu ihrer Herstellung und Flavon-3-carbonsäure-Zwischenprodukte.

In einer Arbeit von A. Costa et al (J.Chem.Soc. Perkin I (1985), 799) über Lithiierung von Flavonen und Umsetzungsreaktionen der lithiierten Produkte sind die 3-Flavoncarbonsäure, die 2'-Methoxy-3-flavoncarbonsäure und die 5,7-Dimethyl-3-flavoncarbonsäure beschrieben worden. Für diese Verbindungen ist jedoch bisher keine pharmakologische Wirksamkeit bekannt.

Der vorliegenden Erfindung liegt die Aufgabe zugrunde, neue Arzneimittel mit entzündungshemmden Eigenschaften zu entwickeln. Ferner liegt der Erfindung die Aufgabe zugrunde, neue Flavon-Verbindungen mit wertvollen pharmakologischen Eigenschaften herzustellen.

Es wurde nun gefunden, daß die erfindungsgemäßen neuen basischen 3-Flavoncarbonsäure-Derivate wertvolle pharmakologische Eigenschaften, insbesondere die Sauerstoffradikalbildung in Entzündungszellen hemmende Eigenschaften, sowie auch Lipoxigenase hemmende und antiphlogistische Eigenschaften besitzen und ein günstiges Wirkungsprofil mit geringer Toxizität und guter Verträglichkeit aufweisen. Aufgrund ihrer Sauerstoffradikalfänger-Eigenschaften wirken die Substanzen entzündungshemmend und eignen sich als Antiinflammatorika und Antiphlogistika zur Behandlung von entzündlichen und allergischen Erkrankungen.

Die vorliegende Erfindung betrifft daher neue basische Flavon-3-carbonsäure-Verbindungen der allgemeinen Formel I

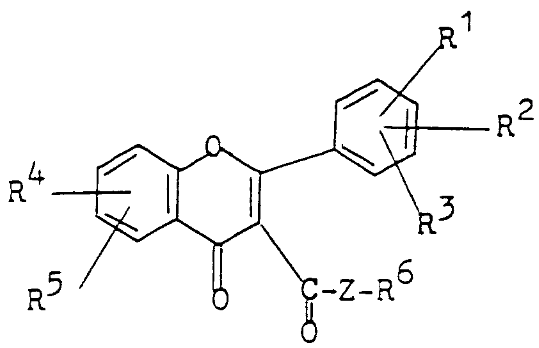

I

worin die Substituenten die folgenden Bedeutungen haben, wobei jedoch solche Substituentenkombinationen, welche aufgrund gegenseitiger sterischer Hinderung nicht möglich sind, ausgenommen sind, und

$R^1$ Wasserstoff, Alkyl mit 1-4 Kohlenstoffatomen, Halogen, Hydroxy, Alkylcarbonyloxy mit 2-5 Kohlenstoffatomen oder eine $R^9$-O-Gruppe, worin $R^9$ für eine Alkyl- oder Alkenylgruppe mit bis zu 20 Kohlenstoffatomen steht, bedeutet,

$R^2$ Wasserstoff, Alkyl mit 1-4 Kohlenstoffatomen, Halogen, Hydroxy, Alkylcarbonyloxy mit 2-5 Kohlenstoffatomen oder eine $R^9$-O-Gruppe, worin $R^9$ obige Bedeutung besitzt, bedeutet, und

$R^3$ Wasserstoff, Alkyl mit 1-4 Kohlenstoffatomen, Halogen, Hydroxy, Alkylcarbonyloxy mit 2-5 Kohlenstoffatomen oder eine $R^9$-O-Gruppe, worin $R^9$ obige Bedeutung besitzt, bedeutet, oder

von den Substituenten $R^1$ bis $R^3$ zwei an benachbarte Kohlenstoffatome gebunden sind und gemeinsam eine Alkylendioxygruppe mit 1-2 Kohlenstoffatomen bedeuten,

wobei jedoch, wenn mehrere der Substituenten $R^1$ bis $R^3$ sauerstoffhaltige Reste darstellen, diese Reste identisch sind,

$R^4$ Wasserstoff, Alkyl mit 1-4 Kohlenstoffatomen, Halogen, Hydroxy, Alkylcarbonyloxy mit 2-5 Kohlenstoffatomen oder eine $R^9$-O-Gruppe, worin $R^9$ obige Bedeutung besitzt, bedeutet, und

$R^5$ Wasserstoff, Alkyl mit 1-4 Kohlenstoffatomen, Halogen, Hydroxy, Alkylcarbonyloxy mit 2-5 Kohlenstoffatomen oder eine $R^9$-O-Gruppe, worin $R^9$ obige Bedeutung besitzt, bedeutet, oder

$R^4$ und $R^5$ an benachbarte Kohlenstoffatome gebunden sind und gemeinsam eine Alkylendioxygruppe mit 1-2 Kohlenstoffatomen bilden,

wobei jedoch, falls beide Substituenten $R^4$ und $R^5$ sauerstoffhaltige Reste darstellen, diese Reste identisch sind, und, falls $R^1$, $R^2$ und/oder $R^3$ Hydroxy oder Alkylcarbonyloxy-Gruppen mit 2-5 Kohlenstoffatomen darstellen, sauerstoffhaltige Reste $R^4$ und/oder $R^5$ mit diesen Gruppen identisch sind,

$R^6$ eine durch Alkyl mit 1-4 Kohlenstoffatomen disubstituierte Aminogruppe, eine Pyridyl- oder Pyrimidylgruppe, eine 1-Benzylpiperidin-4-yl-Gruppe oder die Gruppe a darstellt,

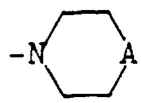

a

worin

A eine Bindung, die Methylengruppe, Sauerstoff oder eine N-$R^7$-Gruppe, bedeutet, worin

$R^7$ Wasserstoff, Alkyl mit 1-4 Kohlenstoffatomen, eine Pyridyl- oder Pyrimidylgruppe oder eine gegebenenfalls durch Alkyl mit 1-4 Kohlenstoffatomen oder Halogen substituierte Benzyl-oder Phenylgruppe bedeutet,

Z eine Y-$(CH_2)_n$-Gruppe bedeutet, worin

Y eine $NR^8$-Gruppe, worin $R^8$ Wasserstoff oder Alkyl mit 1-4 Kohlenstoffatomen ist, oder, falls keiner der Reste $R^1$-$R^5$ Hydroxy oder Alkylcarbonyloxy mit 2-5 Kohlenstoffatomen ist, Y auch Sauerstoff bedeutet, und

n eine ganze Zahl von 2-4 oder, falls $R^6$ eine 1-Benzylpiperidin-4-yl-Gruppe ist, auch Null bedeutet, oder

Z auch eine Bindung bedeutet, falls $R^6$ für eine Gruppe a steht, worin A eine N-$R^7$-Gruppe ist,

sowie N-Oxide und/oder Säureadditionssalze der Verbindungen der Formel I.

Sofern in den Verbindungen der Formel I die Substituenten niedere Alkylgruppen darstellen oder enthalten, können diese gerade oder verzweigt sein und enthalten 1 bis 4, vorzugsweise 1 bis 2 Kohlenstoffatome. Sofern die Substituenten Halogen darstellen oder enthalten, kommen insbesondere Fluor, Chlor oder Brom, vorzugsweise Chlor in Frage.

Die Substituenten $R^1$ bis $R^3$ im B-Ring des Flavon-Gerüstes stellen bevorzugt Wasserstoff oder einen sauerstoffhaltigen Rest dar. Alkoxyreste -$OR^9$ können geradkettig oder verzweigt sein und 1 bis 20, vorzugsweise 1 bis 4 Kohlenstoffatome, enthalten und stellen bevorzugt Methoxyreste dar. Alkenyloxyreste -$OR^9$ können ebenfalls geradkettig oder verzweigt sein und 3 bis 20, vorzugsweise 3 oder 4 Kohlenstoffatome enthalten. Die Doppelbindung ist durch mindestens 1 Kohlenstoffatom von dem Sauerstoffatom getrennt. In niederen Alkylcarbonyloxy-Resten $R^1$-$R^3$ kann die Alkylgruppe geradkettig oder verzweigt sein und enthält 1 bis 4, vorzugsweise 1 bis 2 Kohlenstoffatome. Vorzugsweise stellen Alkanoyloxyreste $R^1$-$R^3$ Acetoxy dar. Vorzugsweise stellen 2 oder 3 der Substituenten $R^1$ bis $R^3$ Methoxy oder Hydroxy dar.

Für die Substituenten $R^4$ und $R^5$ des A-Ringes des Flavon-Gerüstes gilt das vorstehend für die Substituenten des B-Ringes Angegebene ebenfalls. $R^4$ und $R^5$ stellen bevorzugt Wasserstoff dar. Falls sowohl in dem A-Ring wie auch in dem B-Ring sauerstoffhaltige Substituenten enthalten sind, sind diese Substituenten zweckmäßigerweise identisch.

In dem Rest $R^6$ enthaltene Pyridyl- und Pyrimidyl-Gruppen $R^7$ oder $R^8$ sind niemals über ihr Stickstoffatom gebunden. Vorzugsweise sind diese Reste über das Kohlenstoffatom in 2-Stellung angebunden. Falls Z eine Y-$(CH_2)_n$-Gruppe bedeutet, stellt Y vorzugsweise eine $NR^8$-Gruppe dar, worin $R^8$ bevorzugt Wasserstoff bedeutet. Falls $R^8$ für niederes Alkyl steht, kann dieses geradkettig oder verzweigt sein und stellt bevorzugt Methyl dar. Falls $R^6$ durch Alkyl disubstituiertes Amino darstellt, enthalten die in $R^6$ enthaltenen Alkylgruppen 1 bis 4, vorzugsweise 1 bis 2 Kohlenstoffatome. Falls $R^6$ für einen Heterocyclus a steht, stellt A vorzugsweise eine $R^7$-N<Gruppe dar. Falls $R^7$ einen Phenylring darstellt oder enthält, kann dieser unsubstituiert oder mono-, di-oder tri-substituiert sein. Bevorzugt stellt $R^6$ einen durch einen cyclischen Rest, insbesondere einen Pyridylrest substituierten Piperazinring dar.

Erfindungsgemäß werden die neuen Flavon-3-carbonsäure-Verbindungen der Formel I und deren Säureadditionssalze und/oder N-Oxide erhalten, indem man in an sich bekannter Weise

a) zur Herstellung von Verbindungen der allgemeinen Formel Ia

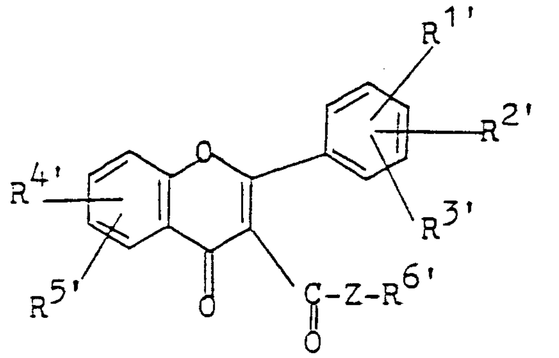

Ia

worin $R^{1'}$, $R^{2'}$, $R^{3'}$, $R^{4'}$ und $R^{5'}$ die für $R^1$, $R^2$, $R^3$, $R^4$ und $R^5$ angegebenen Bedeutungen mit Ausnahme von niederem Alkylcarbonyloxy besitzen, Z obige Bedeutung besitzt und $R^{6'}$ die für $R^6$ angegebene Bedeutung mit Ausnahme solcher Gruppen, worin $R^7$ Wasserstoff bedeutet, besitzt, reaktive Derivate von Säuren der allgemeinen Formel II

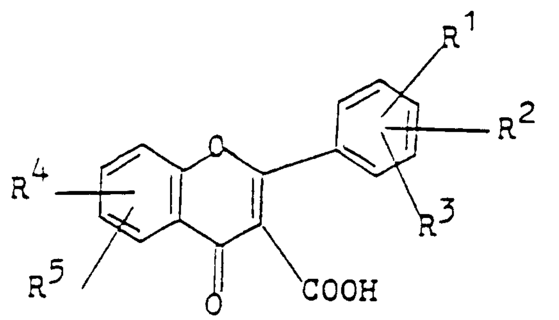

II

worin $R^1$, $R^2$, $R^3$, $R^4$ und $R^5$ obige Bedeutung besitzen, jedoch allfällige Hydroxygruppen mit einer Schutzgruppe versehen sind, mit Verbindungen der allgemeinen Formel III

$H\text{-}Z\text{-}R^{6'}$ III

worin $R^{6'}$ und Z obige Bedeutung besitzen, umsetzt, oder

b) zur Herstellung von Verbindungen der allgemeinen Formel Ib

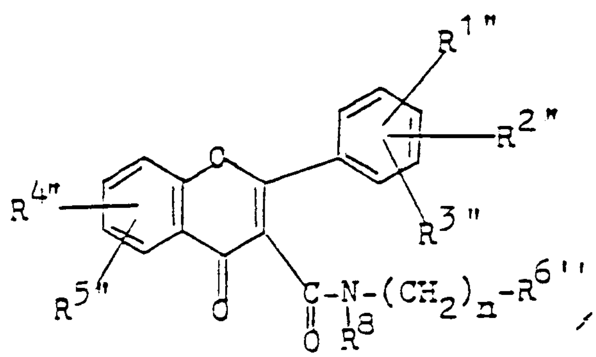

Ib

worin n und $R^8$ obige Bedeutung besitzen, $R^{1''}$, $R^{2''}$, $R^{3''}$, $R^{4''}$ und $R^{5''}$ die für $R^1$, $R^2$, $R^3$, $R^4$ und $R^5$ angegebenen Bedeutung mit Ausnahme von Hydroxy und niederem Alkylcarbonyloxy besitzen, und $R^{6''}$ eine durch niederes Alkyl disubstituierte Aminogruppe oder die oben definierte Gruppe a bedeutet, Verbindungen der allgemeinen Formel IV

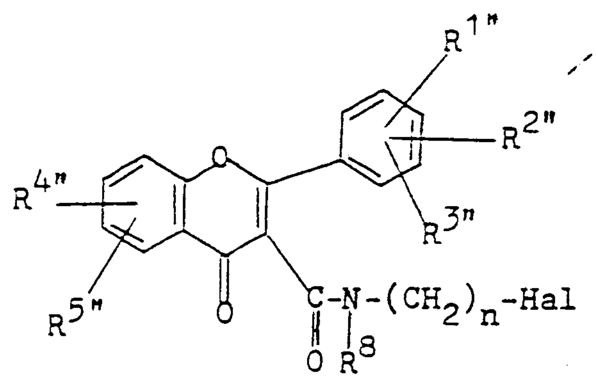

IV

worin $R^{1''}$, $R^{2''}$, $R^{3''}$, $R^{4''}$, $R^{5''}$, n und $R^8$ obige Bedeutung besitzen und Hal Halogen bedeutet, mit Aminen der allgemeinen Formel V

$H\text{-}R^{6''}$ V

worin $R^{6''}$ obige Bedeutung besitzt, umsetzt, und gewünschtenfalls zur Herstellung von Verbindungen der Formel I, worin $R^1$, $R^2$, $R^3$, $R^4$ und/oder $R^5$ niedere Alkylcarbonyloxy-Gruppen bedeuten, in Verbindungen der Formel Ia, worin $R^{1'}$, $R^{2'}$, $R^{3'}$, $R^{4'}$ und/oder $R^{5'}$ freie Hydroxygruppen bedeuten, diese zu niederen Alkylcarbonyloxy-Gruppen acyliert und/oder gewünschtenfalls zur Herstellung von Verbindungen der Formel I, worin $R^1$, $R^2$, $R^3$, $R^4$ und/oder $R^5$ freie Hydroxygruppen bedeuten, in Verbindungen der Formel I, worin $R^1$, $R^2$, $R^3$, $R^4$ und/oder $R^5$ niedere Alkoxygruppen bedeuten, diese in freie Hydroxygruppen überführt und/oder zur Herstellung von Verbindungen der Formel I, worin $R^7$ Wasserstoff bedeutet, aus erhaltenen Verbindungen der Formel I, worin $R^7$ Benzyl bedeutet, die Benzylgruppe abspaltet, und gewünschtenfalls Verbindungen der allgemeinen Formel I zu ihren N-Oxiden oxidiert und/oder in Säureadditionssalze überführt oder die Säureadditionssalze in die freien Verbindungen überführt.

Die Umsetzung von reaktiven Derivaten der Säuren der Formel II mit Aminen oder Alkoholen der Formel III gemäß Verfahrensvariante a) kann nach an sich zur Bildung von Amiden und Estern üblichen Acylierungsmethoden ausgeführt werden. Hierbei werden die Säuren in an sich bekannter Weise durch Überführung in ein reaktionsfähiges Derivat aktiviert. Als reaktionsfähige Säurederivate kommen beispielsweise gemischte Anhydride, z. B. Anhydride mit niederen Alkancarbonsäuren oder niederen Alkylsulfonsäuren, insbesondere Essigsäure oder Methansulfonsäure, oder Säurehalogenide, insbesondere Chloride oder Bromide, in Frage. Es eignen sich beispielsweise Säurederivate der Formel VI

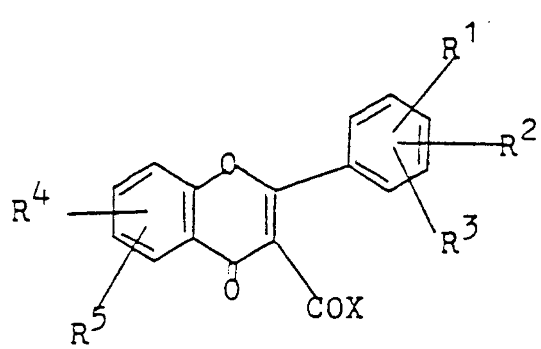

VI

worin $R^1$ bis $R^5$ obige Bedeutung besitzen und X Halogen oder eine Acyloxygruppe -OX′ bedeutet, worin X′ für niederes Alkylcarbonyl oder niederes Alkylsulfonyl steht.

Sofern in den Säuren der Formel II die Substituenten $R^1$, $R^2$, $R^3$, $R^4$, und/oder $R^5$ freie Hydroxygruppen darstellen, müssen diese während der Umsetzung mit den Verbindungen der Formel III auf an sich bekannte Weise durch eine leicht wieder abspaltbare Schutzgruppe geschützt werden. Als Schutzgruppen eignen sich Acylgruppen, z.B. niedere Alkanoylgruppen, beispielsweise Acetylgruppen. Zweckmäßig werden Acylschutzgruppen eingeführt, bevor die Säuren der Formel II in ihre reaktionsfähigen Derivate überführt

werden. Bei der Herstellung gemischter Anhydride mit niederen Alkancarbonsäuren kann die Einführung der Schutzgruppen auch gleichzeitig mit der Anhydridbildung erfolgen.

Die Überführung der freien Säuren der Formel II in reaktive Säurederivate erfolgt auf an sich bekannte Weise. So können Säurehalogenide der Formel VI z.B. durch Umsetzung der Säuren mit einem Säurehalogenid, beispielsweise Phosphortrichlorid, Phosphorpentachlorid, Phosphortribromid, Thionylchlorid oder Oxalylchlorid erhalten werden. Gewünschtenfalls kann die Umsetzung in Gegenwart von Pyridin oder einer anderen tertiären organischen Base durchgeführt werden. Gemischte Säureanhydride können z.B. durch Umsetzung von Säuren der Formel II oder deren Alkalimetallsalzen mit einem entsprechenden organischen Säurechlorid in einem unter den Reaktionsbedingungen inerten organischen Lösungsmittel, beispielsweise einem Halogenkohlenwasserstoff, gegebenenfalls in Gegenwart einer tertiären organischen Base, beispielsweise Pyridin erhalten werden. Hierbei können allfällige freie Hydroxysubstituenten der Säuren der Formel II ebenfalls acyliert und so mit der für die nachfolgende Umsetzung nötigen Schutzgruppe versehen werden.

Die Umsetzung der Säurederivate der Formel VI mit den Verbindungen der Formel III kann in einem unter den Reaktionsbedingungen inerten Lösungsmittel bei Temperaturen zwischen -30 °C und Siedetemperatur des Lösungsmittels, vorzugsweise Temperaturen zwischen -20 °C und Raumtemperatur, erfolgen. Als Lösungsmittel eignen sich halogenierte Kohlenwasserstoffe wie Methylenchlorid oder Chloroform, aromatische Kohlenwasserstoffe wie Benzol oder Toluol, cyclische Äther wie Tetrahydrofuran oder Dioxan oder Gemische dieser Lösungsmittel. Gegebenenfalls kann die Umsetzung in Gegenwart eines säurebindenden Reagenzes durchgeführt werden. Als säurebindende Mittel eignen sich anorganische Basen, insbesondere Alkalimetallcarbonate und -hydroxide und organische Basen, insbesondere tertiäre Niederalkylamine und Pyridine, wie z.B. Triäthylamin, Pyridin, 4-Dimethylaminopyridin oder 4-Pyrrolidinopyridin. Statt einer Fremdbase kann auch ein Überschuß eines Amins der Formel III verwendet werden. Im Überschuß eingesetzte organische Basen können gleichzeitig auch als Lösungsmittel dienen.

Allfällige Acylschutzgruppen von Hydroxysubstituenten werden bei der Umsetzung bzw. im Zuge der Aufarbeitung abgespalten und die Hydroxysubstituenten wieder freigesetzt.

Zweckmäßig kann die Aktivierung der Säure der Formel II durch Umsetzung zu einem gemischten Anhydrid in einem inerten organischen Lösungsmittel, beispielsweise einem halogenierten Kohlenwasserstoff in situ erfolgen und das gebildete gemischte Anhydrid direkt anschließend mit der Verbindung der Formel III weiter umgesetzt werden.

Die Umsetzung der Säure der Formel II mit der Verbindung der Formel III kann zweckmäßigerweise auch in Gegenwart eines aus der Peptidchemie als zur Säureaktivierung für die Amidbildung geeignet bekannten Kopplungsreagenzes durchgeführt werden. Als Beispiele geeigneter Kopplungsreagenzien, welche die Umsetzung dadurch fördern, daß sie mit der Säure in situ unter Bildung eines reaktionsfähigen Säurederivates reagieren, seien insbesondere genannt Alkyl-, vorzugsweise Cycloalkylcarbodiimide, vorzugsweise Dicyclohexylcarbodiimid, Carbonyldiimidazol und N-Niederalkyl-2-halogenpyridiniumsalze, insbesondere Halogenide oder Tosylate, vorzugsweise N-Methyl-2-chlorpyridiniumjodid (siehe z.B. Mukayama in Angew. Chemie 91, 789-812). Die Umsetzung in Gegenwart eines Kopplungsreagenzes kann zweckmäßig bei Temperaturen von -30 °C bis +30 °C unter Benutzung von Lösungsmitteln wie halogenierten Kohlenwasserstoffen und/oder aromatischen Lösungsmitteln gegebenenfalls in Gegenwart eines säurebindenden Amins durchgeführt werden.

Die Umsetzung von Verbindungen der Formel IV mit Aminen der Formel V gemäß Verfahrensvariante b) kann auf an sich bekannte Weise unter zur Alkylierung von Aminen üblichen Bedingungen erfolgen. Der Halogenrest Hal in den Verbindungen der Formel IV kann für Chlor, Brom oder Jod, vorzugsweise Brom, stehen. Zweckmäßig wird die Umsetzung in einem unter den Reaktionsbedingungen inerten organischen Lösungsmittel bei Temperaturen zwischen Raumtemperatur und Siedetemperatur des Lösungsmittels durchgeführt. Als Beispiel geeigneter Lösungsmittel seien Dimethylformamid, aromatische Kohlenwasserstoffe wie Benzol oder Toluol oder halogenierte Kohlenwasserstoffe wie Dichlormethan genannt. Gewünschtenfalls kann die Reaktion unter Zusatz einer organischen oder anorganischen Base durchgeführt werden. Es kann jedoch auch ein Überschuß des Amins der Formel V verwendet und dieses als interne Base benutzt werden. Falls Chloride oder Bromide der Formel IV eingesetzt werden, können zur Beschleunigung der Reaktion Jodidionen in Form eines Jodidsalzes, beispielsweise Kaliumjodid, zugesetzt werden.

Sofern die erhaltenen Verbindungen der Formel Ia freie Hydroxygruppen $R^{1'}$, $R^{2'}$, $R^{3'}$, $R^{4'}$ und/oder $R^{5'}$ enthalten, können diese gewünschtenfalls auf an sich bekannte Weise zu niederen Alkylcarbonyloxy-Gruppen acyliert werden. Die Acylierung erfolgt nach zur Esterbildung durch Acylierung üblichen Methoden, z.B. durch Umsetzung mit reaktiven Derivaten der Säuren der Formel VII

$R^{10}$-CO-OH VII

worin $R^{10}$ niederes Alkyl bedeutet. Als reaktionsfähige Derivate eignen sich insbesondere Halogenide und Anhydride der Säuren der Formel VII. Die Umsetzung kann unter den vorstehend für die Umsetzung von reaktiven Derivaten der Säuren der Formel II mit Verbindungen der Formel III angegebenen Bedingungen erfolgen.

Verbindungen der Formel I, worin $R^1$, $R^2$, $R^3$, $R^4$ und/oder $R^5$ freies Hydroxy bedeuten, können zweckmäßig aus entsprechenden Verbindungen der Formel I, worin $R^1$, $R^2$, $R^3$, $R^4$ und/oder $R^5$ niederes Alkoxy, insbesondere Methoxy, bedeuten, durch Ätherspaltung hergestellt werden. Die Freisetzung der Hydroxygruppen kann nach an sich zur Phenolätherspaltung üblichen Methoden erfolgen. Als günstig erweist sich beispielsweise die Ätherspaltung durch Behandeln der Verbindungen mit Lewissäuren, insbesondere Bortribromid oder auch Trimethylsilyljodid in einem unter den Reaktionsbedingungen inerten Losungsmittel, beispielsweise einem halogenierten Kohlenwasserstoff bei Temperaturen zwischen -100 °C und +50 °C, vorzugsweise zwischen 0 °C und Raumtemperatur.

Zur Herstellung von Verbindungen der Formel I, worin $R^7$ Wasserstoff bedeutet, kann aus entsprechenden Verbindungen der Formel I, worin $R^7$ eine gegebenenfalls substituierte Benzylgruppe bedeutet, diese in an sich bekannter Weise hydrogenolytisch abgespalten werden.

Die Hydrogenolyse kann mit Wasserstoff unter Verwendung von Palladium/Kohle als Katalysator in einem organischen polaren Lösungsmittel, beispielsweise einem niederen Alkohol in Gegenwart einer unter den Reaktionsbedingungen stabilen Säure, beispielsweise einer Halogenwasserstoffsäure erfolgen.

Die Verbindungen der Formel I können in an sich bekannter Weise zu ihren entsprechenden N-Oxiden oxidiert werden. Die Oxidation kann z.B. mit Wasserstoffperoxid oder vorzugweise mit organischen Persäuren in einem unter den Reaktionsbedingungen inerten organischen Lösungsmittel, beispielsweise nach den in Chem. Rev. 68, 747 (1968) beschriebenen Verfahren, erfolgen. Insbesondere eignen sich als Oxidationsmittel Perbenzoesäuren, wie z.B. 3-Chlorperbenzoesäure. Als Lösungsmittel eignen sich z.B. halogenierte Kohlenwasserstoffe wie Dichlormethan, Sofern die Verbindungen mehrere basische Zentren enthalten, können die Stickstoffatome aller diese Zentren oxidiert werden.

Die Verbindungen der Formel I können auf an sich bekannte Weise aus dem Reaktionsgemisch isoliert und gereinigt werden. Säureadditionssalze können in üblicher Weise in die freien Basen überführt werden und diese gewünschtenfalls in bekannter Weise in pharmakologisch verträgliche Säureadditionssalze überführt werden. Sofern die Verbindungen der Formel I mehrere basische Zentren enthalten, können sie Säureadditionssalze mit nur einem oder mit mehreren äquivalenten Säuren bilden.

Als pharmakologisch annehmbare Säureadditionssalze der Verbindungen I eignen sich beispielsweise deren Salze mit anorganischen Säuren wie Chlorwasserstoffsäure, Bromwasserstoffsäure, Schwefelsäure, Phosphorsäure oder organischen Säuren wie Weinsäure, Benzoesäure, Essigsäure, Milchsäure, Zitronensäure, Maleinsäure, Fumarsäure, Äpfelsäure, Niederalkansulfonsäuren, Benzolsulfonsäure oder Toluolsulfonsäuren.

Die als Ausgangsprodukte eingesetzten Flavon-3-carbonsäuren der Formel II, worin Hydroxysubstituenten gewünschtenfalls durch Acyl, insbesondere niederes Alkylcarbonyl, geschützt sein können, und ihre Salze mit Ausnahme der 3-Flavoncarbonsäure, der 2'-Methoxy-3-flavoncarbonsäure und der 5,7-Dimethyl-3-flavoncarbonsäure sind in der Literatur bisher noch nicht beschrieben worden und stellen neue wertvolle Zwischenprodukte für die Herstellung von pharmakologisch aktiven Verbindungen, beispielsweise den Verbindungen der Formel I dar.

Säuren der Formel IIa

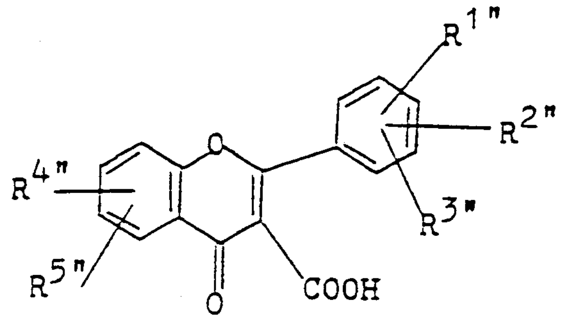

IIa

worin $R^{1''}$, $R^{2''}$, $R^{3''}$, $R^{4''}$ und/oder $R^{5''}$ obige Bedeutung besitzen, können auf an sich bekannte Weise ausgehend von entsprechenden Flavonen der Formel VIII

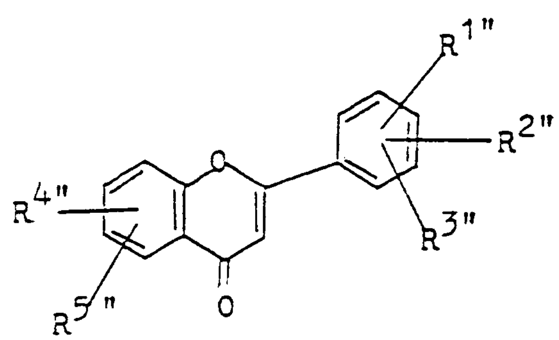

VIII

worin worin $R^{1''}$, $R^{2''}$, $R^{3''}$, $R^{4''}$ und $R^{5''}$ obige Bedeutung besitzen, erhalten werden.

Die Flavone der Formel VIII werden durch Umsetzung mit nukleophilfreien organischen Lithiumbasen zunächst in 3-Stellung lithiiert und durch Behandeln des lithiierten Zwischenproduktes der Formel IX

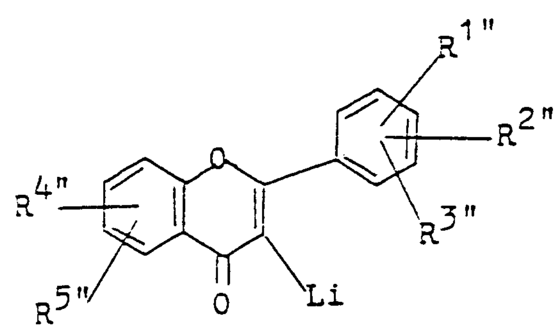

IX

worin $R^{1''}$, $R^{2''}$, $R^{3''}$, $R^{4''}$ und $R^{5''}$ obige Bedeutung besitzen, mit Kohlendioxid wird das Lithium anschließend durch die Carboxylgruppe ersetzt. Als Lithiierungsmittel geeignete organische Lithiumbasen sind insbesondere Lithiumaminbasen vorzugsweise Lithiumdiisopropylamid oder auch Lithiumtetramethylpiperidid oder Lithiumhexamethyldisilazid. Die Lithiierung wird in einem unter den Reaktionsbedingungen inerten Lösungsmittel beispielsweise einem cyclischen Äther wie Tetrahydrofuran bei Temperaturen zwischen -100 °C und -30 °C, vorzugsweise Temperaturen zwischen -90 °C und -50 °C, durchgeführt. Die erhaltenen 3-Lithioflavin-Verbindungen werden durch Behandeln der Reaktionslösung mit Kohlendioxid und anschließendes Ansäuern direkt zu den Säuren der Formel IIa weiterverarbeitet. Zweckmäßigerweise findet die Reaktion mit Kohlendioxid bei Temperaturen zwischen -100 °C und -30 °C in einem unter den Reaktionsbedingungen inerten Lösungsmittel, beispielsweise einem cyclishchen Äther wie Tetrahydrofuran statt. Das Kohlendioxid kann in Form von zerstoßenem Trockeneis in die Reaktionslösung gegeben oder in Form von Kohlendioxidgas eingeleitet werden.

Zur Herstellung von Säuren der Formel II, worin die Substituenten $R^1$, $R^2$, $R^3$, $R^4$ und/oder $R^5$ Hydroxy bedeuten, können entsprechende Methoxy-substituierte Säuren der Formel II demethyliert werden. Die Demethylierung kann nach an sich zur Phenylätherspaltung üblichen Methoden, beispielsweise unter den vorstehend für die Herstellung von freien Hydroxysubstituenten enthaltenden Verbindungen der Formel I aus entsprechenden Nieder-Alkoxy-substituierten Verbindungen der Formel I angegebenen Bedingungen durchgeführt werden.

Zur Herstellung von Säuren der Formel II, worin Hydroxy-Substituenten $R^1$, $R^2$, $R^3$, $R^4$ und/oder $R^5$ durch niedere Alkanoylgruppen, vorzugsweise Acetylgruppen geschützt sind, können die entsprechenden durch freie Hydroxygruppen substituierten Säuren durch Umsetzung mit Halogeniden oder Anhydriden der entsprechenden niederen Alkancarbonsäuren, beispielsweise Alkancarbonsäuren mit 1 bis 4 Kohlenstoffatomen in der Alkankette, auf an sich bekannte Weise acyliert werden.

Verbindungen der Formel IV sind in der Literatur bisher noch nicht beschrieben worden und stellen wertvolle Zwischenprodukte zur Herstellung von pharmakologisch aktiven Verbindungen, beispielsweise

Verbindungen der Formel I dar.

Verbindungen der Formel IV können erhalten werden, indem man reaktionsfähige Derivate von Säuren der Formel IIa mit Aminoalkoholen der Formel X

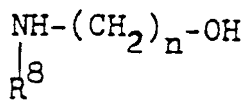

X

worin $R^{10}$ und n obige Bedeutung besitzen, umsetzt zu Verbindungen der Formel XI

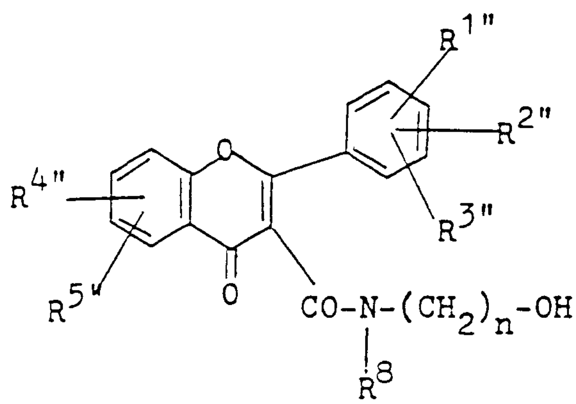

XI

worin $R^{1''}$, $R^{2''}$, $R^{3''}$, $R^{4''}$, $R^{5''}$, $R^8$ und n obige Bedeutung besitzen, und anschließend durch Umsetzen mit Phosphorhalogeniden die Hydroxygruppe in an sich bekannter Weise durch Halogen ersetzt. Die Umsetzung der reaktionsfähigen Derivate der Säuren der Formel IIa mit den Aminoalkoholen der Formel X kann nach an sich zur Amidbildung durch Aminoacylierung üblichen Methoden, beispielsweise unter den vorstehend für die Umsetzung der reaktionsfähigen Derivate der Säuren der Formel II mit den Verbindungen der Formel III angegebenen Reaktionsbedingungen durchgeführt werden.

Die Flavon-Verbindungen der Formel VIII sind bekannt oder können nach an sich bekannten Methoden hergestellt werden. Beispielsweise können Flavone der Formel VIII auf an sich bekannte Weise erhalten werden, indem 2-Hydroxyacetophenon-Verbindungen der Formel XII

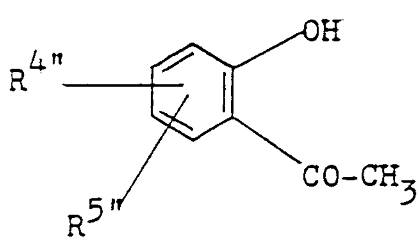

XII

worin $R^{4''}$ und $R^{5''}$ obige Bedeutung besitzen, zunächst mit Benzoylchlorid-Verbindungen der Formel XIII

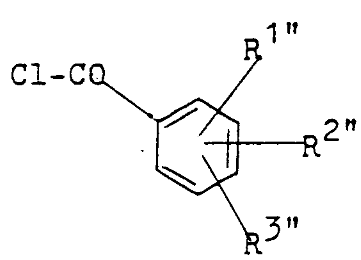

XIII

worin $R^{1''}$, $R^{2''}$, und $R^{3''}$ obige Bedeutung besitzen, umgesetzt werden zu Benzoesäureester-Verbindungen der Formel XIV

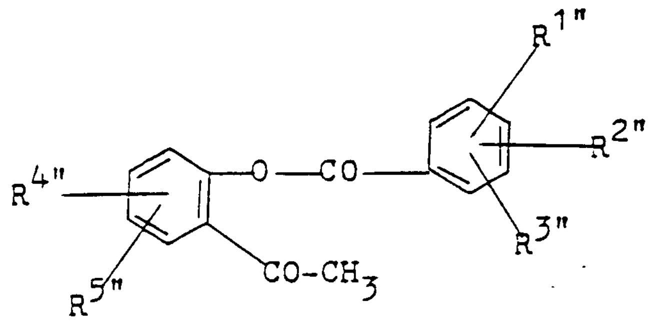

XIV

worin $R^{1''}$, $R^{2''}$, $R^{3''}$, $R^{4''}$ und $R^{5''}$ obige Bedeutung besitzen, und diese anschließend durch Behandlung mit starken Basen umgelagert werden zu Verbindungen der Formel XV

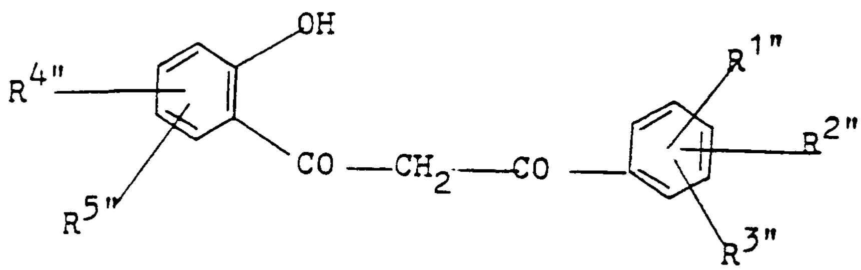

XV

worin $R^{1''}$, $R^{2''}$, $R^{3''}$, $R^{4''}$ und $R^{5''}$ obige Bedeutung besitzen, welche ihrerseits unter Wasserabspaltung zu den Flavonen der Formel VIII cyclisiert werden.

Die Umsetzung der 2-Hydroxyacetophenone der Formel XII mit den Benzoylchloriden der Formel XIII wird in einem unter den Reaktionsbedingungen inerten Lösungsmittel in Gegenwart einer zum Abfangen der gebildeten Chlorwasserstoffsäure ausreichenden Menge einer nicht nukleophilen organischen Base bei Temperaturen zwischen Raumtemperatur und Rückflußtemperatur des Lösungsmittels, vorzugsweise Temperaturen zwischen Raumtemperatur und 80 °C, durchgeführt. Als organische Basen eignen sich tertiäre Amine wie Pyridin oder Triäthylamin, welche gleichzeitig auch als Lösungsmittel dienen können. Als weitere organische Lösungsmittel eignen sich halogenierte Kohlenwasserstoffe. Die erhaltenen Benzoesäureester werden anschließend auf an sich bekannte Weise in einer Baker-Venkataraman-Umlagerung in die Diketonverbindungen der Formel XV überführt. Die Umlagerung kann in einem unter den Reaktionsbedinungen inerten Lösungsmittel, vorzugsweise Pyridin, durch Behandeln mit einer starken Base, beispielsweise Alkalimetallhydroxid, welches in der Reaktionslösung suspendiert wird, bei Temperaturen zwischen ca. 0 °C und Raumtemperatur erfolgen. Die anschließende Cylcisierung der Diketonverbindungen der Formel XV wird zweckmäßigerweise in saurem Medium in Gegenwart eines wasserabspaltenden Reagenzes bei erhöhter Temperatur, vorzugsweise Siedetemperatur des des Reaktionsgemisches durchgeführt. Als günstig erweist sich beispielsweise die Cyclisierung in einer Eisessig/Schwefelsäurelösung.

Flavon-Verbindungen der Formel VIII, worin worin $R^{1''}$, $R^{2''}$, $R^{3''}$, $R^{4''}$ und/oder $R^{5''}$ eine $R^9$-O-Gruppe bilden, können gewünschtenfalls auch durch Verätherung entsprechender hydroxysubstituierter Flavone erhalten werden, indem man diese mit Verbindungen der Formel XVI

$R^9X$ XVI

worin $R^9$ und Hal obige Bedeutung besitzen, unter zur Phenolätherbildung geeigneten Bedingungen umsetzt. Die Verätherung von Hydroxyflavonen wird insbesondere zur Einführung von längerkettigen $R^9$-O-Resten verwendet. Die Hydroxyflavone sind bekannt und/oder können gewünschtenfalls durch Demethylierung entsprechender Methoxyflavone hergestellt werden.

Die Verbindungen der Formel III sind bekannt oder können nach an sich bekannten Methoden erhalten werden.

Die Verbindungen der Formel I und ihre pharmakologisch akzeptablen Säureadditionssalze besitzen interessante pharmakologische Eigenschaften, insbesondere entzündungshemmende, antiallergische und ödemhemmende Eigenschaften und zeichnen sich durch ein günstiges Wirkungsprofil mit oraler Wirksamkeit sowie guter Veträglichkeit und geringer Toxizität aus.

So besitzen die Verbindungen insbesondere eine hemmende Wirkung gegenüber der Bildung von Sauerstoffradikalen aus Entzündungszellen. Es ist bekannt, daß aktivierte Entzündungszellen Sauerstoffradikale und sauerstoffhaltige Moleküle mit hoher oxidativer Potenz freisetzen. Alle diese reaktiven Sauerstoffverbindungen werden im folgenden mit dem Begriff Sauerstoffradikale bezeichnet. Sauerstoffradikale sind insbesondere bei akuten und chronischen Entzündungen, der Toxizität von Fremdsubstanzen verschiedenster Art und bei Röntgen- oder radioaktiver Strahlung verantwortlich für die Alteration von Zell- oder Gewebsproteinen und Lipiden. Die sauerstoffradikalbildende Aktivität von Entzündungszellen hemmende Substanzen wirken daher vorbeugend und hemmend gegenüber mit Entzündungserscheinungen verbundenen Krankheitszuständen und vermögen die Bildung von durch Entzündungen hervorgerufenen Zellschädigungen zu hemmen.

Die Verbindungen zeigen ferner eine ausgeprägte und spezifische Lipoxygenase-Hemmung. Das Lipoxygenase-Enzym steuert die Biosynthese von biologisch aktiven Mediatoren vom Leukotrien-Typ. Bekannterweise sind Leukotriene maßgeblich an der Induzierung von entzündlichen und allergischen Reaktionen im menschlichen Körper beteiligt, beispielsweise auch bei der Entstehung von asthmatischen Krankheiten. Sie können auch negative Einwirkungen auf das Herzkreislaufgeschehen haben. Lipoxygenasehemmende Substanzen wirken vorbeugend und hemmend gegenüber den durch Mediatoren vom Leukotrien-Typ hervorgerufenen Krankheitssymptomen, welche z.B. bei rheumatischen, allergischen und asthmatischen Erkrankungen auftreten können. Sie sind daher in die Behandlung von Krankheiten dieses Formenkreises nützlich.

Darüber hinaus besitzen die Verbindungen auch ödemhemmende Eigenschaften.

Die Sauerstoffradikalbildung in Entzündungszellen hemmenden und die Lipoxygenase hemmenden Eigenschaften der Verbindungen der Formel I können in pharmakologischen Standardtests in vitro nachgewiesen werden. Die ödemhemmenden Eigenschaften werden zweckmäßig in pharmakologischen Standardtests an Tieren nachgewiesen.

Beschreibung der Testmethoden

1. Zur Bestimmung der hemmenden Wirkung auf die sauerstoffradikalbildende Aktivität von Entzündungszellen wird die in vitro-Hemmung der Freisetzung von Sauerstoffradikalen aus aktivierten peritonalen Exsudatzellen ( = PEC) von Mäusen im Chemilumineszenz(CL)-Test nach der Methode von Müller-Peddinghaus (Int. J. Immunopharmac. 6, 455-466 (1984)) bestimmt. Hierbei wird die durch Reaktion der in einer aktivierten PEC-Suspension enthaltenen Sauerstoffradikale mit dem Chemilumineszenzverstärker Lucigenin (= 10,10′-Dimethyl-bis-9,9′-acridiniumnitrat) erzeugte Chemilumineszenz gemessen. Die Intensität der Chemilumineszenz ist ein Maß für den Gehalt an Sauerstoffradikalen in der Suspension und somit den Grad der Aktivität der Entzündungszellen.

Dabei werden drei funktionell verschiedene Parameter erfaßt, die spontane CL, die induzierte CL und die Peak CL. Die spontane CL und die induzierte CL auf der einen Seite und die Peak CL auf der anderen Seite unterscheiden sich dadurch voneinander, daß bei den beiden ersten Parametern die Testsubstanzen während einer 30-minütigen Vorinkubationsperiode im Kontakt mit den Zellen stehen bevor der Entzündungsreiz gesetzt wird, während bei der Peak CL zunächst die Zellen stimuliert werden und im Gipfel ihrer Aktivität die Testsubstanz zugegeben wird. Die Messung der spontanen CL erfaßt somit den Einfluß der Testsubstanzen auf die relative Ruheaktivität von Entzündungszellen, die induzierte CL repräsentiert die Wirkung der Testsubstanzen auf die Aktivierbarkeit der Zellen und die Hemmung der Peak CL gibt den Effekt der Testsubstanzen auf die maximale Aktivität der Entzündungszellen an.

1.1 Herstellung der in dem Test verwendeten PEC-Suspension. Zur Induzierung der PEC-Bildung werden weiblichen Mäusen von 20-25 g Körpergewicht je 2 ml einer 3 %-igen Lösung von Thioglykolat in steriler isotoner Kochsalzlösung i.p. injiziert. 24 Std. nach der Thioglycolatinjektion werden die Zellen, welche vorwiegend aus polymorphkernigen Leukozyten bestehen, durch Ausspülen der Bauchhöhle gewonnen und zweimal gewaschen. Als Zellkulturmedium wird eine unter der Bezeichnung RPMI 1640 (pH 7,2) im Handel befindliche Lösung (Hersteller Flow Laboratories, Meckenheim) eingesetzt, welches 10 % foetales Kälberserum (FCS, Hersteller Seromed GmbH, München) 18 µmol/l Natriumbikarbonat und 2 µmol/l L-Glutamin enthält. Die Zellpellets von jeweils 4 Tieren werden im Zellkulturmedium resuspendiert, zusammengefaßt und die Suspension mit Zellkulturmedium auf 2 x $10^6$ Zellen pro ml eingestellt. Die Zellsuspensionen werden bis zur Verwendung

am gleichen Tage eisgekühlt aufbewahrt.

1.2 Herstellung der zur Aktivierung verwendeten Komplement-opsonierten Zymosan (= Zy $C_3$b)-Suspension. 200 mg Zymosan (Glykoproteinmischung, isoliert aus den Zellwänden von Bierhefe, Sacchomyces cerivisiae, Hersteller Sigma Chemical Co., München) werden in 10 ml Phosphatpufferlösung nach Dulbecco (Hersteller Flow Laboratories, Meckenheim) suspendiert, gut gerührt und 30 min lang im Wasserbad auf 100 °C erhitzt. Nach dem Abkühlen wird 5 min lang bei 4 °C und 600 x g zentrifugiert und der Überstand abdekantiert. Das Sediment wird in 50 ml Humanserum resuspendiert und 30 min lang bei 37 °C im Schüttelwasserbad inkubiert. Anschließend wird 5 min lang bei 600 x g und 4 °C zentrifugiert, der Überstand dekantiert und das Sediment mit Phosphatpufferlösung gewaschen. Aus dem Sediment wird durch Verdünnen mit Phosphatpufferlösung die Gebrauchssuspension von 8 x $10^{-3}$ g Zy$C_3$b /ml hergestellt (Müller-Peddinghaus et al, Zbl. Vet. Med. B. 30, 559-575).

1.3 Chemilumineszenz (CL)-Messung (Peak-CL). Die PEC-Suspension wird in Proben von je 100 µl aufgeteilt, in Reagenzröhrchen gegeben und diese werden 10 min bei einer Temperatur von 37 °C im Meßgerät (Chemilumineszenzanalysator, Laboratorium Prof. Berthold, Wildbad) inkubiert. Anschließend werden zur Verstärkung der Chemilumineszenz 100 µl Lucigeninlösung zugegeben (Endkonzentrat 1,54 x $10^{-4}$ mol/l) und weitere 16 min inkubiert. Die Zellen werden sodann durch Zugabe von 100 µl der unter 1.2 hergestellten Zy$C_3$b-Suspension + 100 µl Phosphatpufferlösung aktiviert. Die maximale Aktivität der Zellen ist nach 16 min erreicht. Zu diesem Zeitpunkt werden 100 µl Testsubstanz (enthaltend $10^{-4}$ Mol Substanz/l) zugegeben und die durch die Substanzen auftretende Erniedrigung des Chemilumineszenzsignals im Vergleich zu Kontrollen bestimmt. Das Ergebnis wird als % Hemmung der Sauerstoffradikalfreisetzung bestimmt.

1.4 Chemilumineszenz-Messung (spontane CL und induzierte CL). Die Bestimmung dieser beiden Parameter folgt dem gleichen Testprinzip wie die Messung der Peak-CL mit dem Unterschied, daß 100 µl PEC-Suspension gemeinsam mit 100 µl Testsubstanzlösung 30 min lang bei 37 °C im Meßgerät vorinkubiert werden. Im Anschluß an diese Vorinkubationsphase werden 100 µl Lucigenin zugegeben und es wird innerhalb der folgenden 16 min Inkubationsphase mit zwei Meßpunkten der Parameter spontane CL erfaßt. Danach werden 100 µl Zy$C_3$b + 100 µl Phosphatpuffer zugegeben, und es wird in den folgenden 24 min der Zellaktivierung mit drei Meßpunkten der Parameter induzierte CL erfaßt.

Alle Messungen sind Doppelbestimmungen. Die Parameter spontane CL und induzierte CL werden am selben Tag mit zwei separaten Zellpools durchgeführt.

2. Zur Untersuchung der lipoxygenasehemmenden Eigenschaften wird die Hemmwirkung der Substanzen auf die Biosynthese von Leukotrien $B_4$ (= $LTB_4$) aus Arachidonsäure durch aktivierte Entzündungszellen in vitro bestimmt.

Leukotrien $B_4$, eine Dihydroxyfettsäure, welche sowohl bei Entzündungen wie bei allergischen Reaktionen eine wichtige Rolle spielt, wird ausgehend von Arachidonsäure nach Oxidation durch die Wirkung das Enzyms Lipoxygenase oxidativ gebildet.

$LTB_4$-Synthese und -Inhibitions-Test.

Polymorphkernige Leukozyten (PMNL) werden auf an sich bekannte Weise nach der Methode von Carlson und Kaneko (Proc.Soc. Exp. Med. 142, (1973) 853-856) gereinigt. Die Zellen werden in einem Inkubationsmedium (pH 7,4) suspendiert, das 150 mM NaCl, 4mM KCl, 2,5 mM $Na_2HPO_4$, 3,5 mM $KH_2PO_4$, 0,75 mM $CaCl_2$ und 5 mM Glucose beinhaltet. Jede Probe enthält in einem Gesamtvolumen von 2 ml Medium 3 x $10^7$ Zellen und wird über einen Zeitraum von 30 min bei 37 °C mit 10 µl Testsubstanzlösung ($10^{-4}$ Mol/l Testsubstanz gelöst in Dimethylsulfoxid oder Äthanol) vorinkubiert. Danach folgt die Zugabe von Ca-Ionophor A 23187 (Hersteller Sigma Chem. Co., München) (10 mmol/l Endkonzentration) und nach weiteren 20 min werden Arachidonsäure (Hersteller Sigma Chem. Co.) (1,5 x $10^{-5}$ mol/l Endkonzentration) und Kalzium (2 x $10^{-3}$ mol/l Endkonzentration) zugegeben.

Nach weiteren 10 min bei 37 °C werden die Zellen abzentrifugiert und der Überstand zweimal mit Diäthyläther extrahiert. Die vereinigten Extrakte werden im Vakuum zur Trockene eingedampft und der Rückstand in 0,5 ml 30 % wäßrigem Methanol aufgenommen und mit Hilfe der Hochleistungsflüssigkeitschromatographie mit Phasenumkehr (= reversed phase-HPLC) analysiert. Die stationäre Phase (= Trennmedium) besteht aus Octadecylgruppen chemisch gebunden enthaltendem Kieselgel (= RSIL Cl8 HP; Hersteller Autech, Darfield, IL.). Das Lösungsmittelgemisch ist Methanol : Wasser : Essigsäure (67:33: 0,1; v/v/v), das mit $NH_3/H_2O$ auf einen pH-Wert von 6,2 eingesgtellt ist.

Die Fließgeschwindigkeit beträgt 1 ml/min. Die Messung von $LTB_4$ erfolgt bei 280 nm mit einem Spectro-Monitor III (LDC-Milton Ray, Riviera Beach, Florida) und die Daten werden aufgenommen und integriert von einem Varian 4270 Integrator (Varian, Palo Alto, Cal.).

Die $LTB_4$-Signale werden mit denen der Kontrolle (Zellinkubation mit Lösungsmittel) verglichen und als Prozent Inhibiton der $LTB_4$-Synthese gegenüber den Kontrollen angegeben.
3. Bestimmung der minimalen toxischen Dosis.
Männlichen Mäusen von 20-25 g Gewicht werden per os Maximaldosen von 300 mg/kg der Testsubstanz verabreicht. Die Tiere werden drei Stunden lang sorgfältig auf Toxizitätssymptome beobachtet. Über einen Zeitraum von 24 Stunden nach der Applikation werden zusätzlich alle Symptome und Todesfälle registriert. Begleitsymptome werden ebenfalls beobachtet und registriert. Wenn Tod oder starke toxische Symptome beobachtet werden, werden weiteren Mäusen zunehmend geringere Dosen verabreicht. Die niedrigste Dosis, welche Tod oder starke toxische Symptome hervorruft, wird als minimale toxische Dosis angegeben.

Die folgende Tabelle A gibt nach den vorstehend beschriebenen Testmethoden erhaltene Ergebnisse wieder. Die für die Verbindungen der Formel I angegebenen Beipielsnummern beziehen sich auf die nachstehenden Herstellungsbeispiele.

Tabelle A

| Testsubstanz Beisp. Nr. | Hemmwirkung in vitro auf Sauerstoffradikalfreisetzung im Chemilumineszenztest % Hemmung Peak CL | spont.CL | ind.CL | Lipoxygenasehemmung in vitro % Hemmung der $LTB_4$-Synth. bei $10^{-4}$ Mol/l | Minimale toxische Dosis mg/kg Maus p.o. |
|---|---|---|---|---|---|
| 1c | 47 | 49 | 39 | 52 | >300 |
| 2b | 58 | 67 | 54 | 92 | >300 |
| 3b | 61 | 94 | 83 | 8 | 300 |
| 4e | 76 | 95 | 82 | 10 | 300 |
| 5 | 12 | 49 | 25 | | >300 |
| 7a | 68 | 75 | 65 | 38 | >300 |
| 7b | 44 | 61 | 50 | 42 | >300 |
| 14e | 99 | 98 | 99 | 86 | >300 |
| 18b | 41 | 30 | 50 | | 300 |
| 20b | 27 | 72 | 43 | | 100 |
| 22b | 25 | 67 | 43 | | 300 |
| 23 | 63 | 95 | 88 | | 300 |
| 24 | 1 | 55 | 14 | 46 | >300 |
| 26 | 10 | 13 | 3 | 43 | |
| 28 | 5 | 54 | 29 | | >300 |
| 29 | 75 | 65 | 74 | 97 | |
| 30 | 85 | 85 | 85 | 44 | >300 |
| 32b | 56 | 71 | 65 | 25 | >300 |
| 34 | 25 | 54 | 55 | 25 | |
| 35 | | | | 100 | |
| 39b | 99 | 99 | 99 | 93 | |
| 42b | 99 | 99 | 100 | 100 | |
| 46 | 11 | 52 | 25 | | >300 |
| 47 | 70 | 78 | 76 | | 100 |
| 49 | 41 | 86 | 64 | | 300 |
| 53 | 77 | 73 | 83 | 77 | |
| 54 | 72 | 91 | 83 | 44 | >300 |
| 56 | 37 | 52 | 70 | | |
| 59 | 84 | 97 | 94 | | >300 |

Die antiödematösen Eigenschaften der Verbindungen der Formel I können durch ihre inhibierenden Wirkungen gegenüber durch Carrageenin-Injektionen in der Rattenpfote verursachter lokaler Ödembildung nachgewiesen werden.

Beschreibung der Testmethode zur Bestimmung der Hemmwirkung auf das Carrageenin-Pfotenödem in der Ratte nach der Methode von Winter et al (Proc.Soc.Exp.Biol.Med. 111, (1962) 544-547).

Es werden männliche Wistar-Ratten mit einem Körpergewicht von etwa 120-140 g verwendet. Eine Dosis der Testsubstanz wird suspendiert in einem Volumen von 0,5 ml pro 100 g Körpergewicht einer 1%-igen Tylose[R]-Lösung (=Methylcellulose) mittels einer Schlundsonde per os verabreicht oder i.p. injiziert. Eine Kontrollgruppe erhält nur die Tylose-Lösung. Eine Stunde später werden zum Setzen der Entzündung als Irritanz 0,1 ml einer 1%-igen Suspension von Carrageenin (Satiagum E[R]) in isotonischer Kochsalzlösung intraplantar in die rechte Hinterpfote injiziert. In die linke Hinterpfote wird ein gleiches Volumen an isotonischer Kochsalzlösung injiziert. Sowohl vor als auch 2 Stunden nach Applikation des Irritanz wird das Volumen der einzelnen Rattenpfoten plethysmometrisch gemessen und die Pfotenvolumenschwellung nach Carrageneengabe im Vergleich zur nur mit Kochsalzlösung behandelten Pfote bestimmt. Es wird die durch die Testsubstanzen bewirkte Hemmung der Ödembildung bei den behandelten Tieren im Vergleich zu den Tieren der unbehandelten Kontrollgruppe in % angegeben.

Die folgende Tabelle B gibt nach der vorstehend beschriebenen Methode mit Verbindungen der Formel I erhaltene Ergebnisse wieder.

## Tabelle B

| Testsubstanz Beispiel Nr. | Dosis µmol/kg | % Hemmung des Carrageneen Pfotenödems |
|---|---|---|
| 1c | 100 p.o. | 44 |
| 1c | 215 i.p. | 55 |
| 2b | 100 p.o. | 25 |
| 4e | 215 p.o. | 50 |
| 4e | 215 i.p. | 54 |
| 6b | 215 i.p. | 75 |
| 7b | 215 p.o. | 40 |
| 10b | 215 p.o. | 30 |
| 10b | 215 i.p. | 35 |
| 14e | 215 i.p. | 33 |
| 16a | 100 p.o. | 20 |
| 25 | 215 p.o. | 25 |
| 25 | 215 i.p. | 58 |
| 34 | 215 p.o. | 30 |
| 39b | 215 i.p. | 62 |
| 36 | 215 p.o. | 30 |
| 52 | 215 i.p. | 53 |
| 53 | 215 i.p. | 50 |
| 54 | 215 i.p. | 40 |

Als Heilmittel können die Verbindungen der Formel I, ihre N-oxide und ihre physiologisch verträglichen Säureadditionssalze mit üblichen pharmazeutischen Hilfsstoffen in galenischen Zubereitungen wie z.B. Tabletten, Kapseln, Suppositorien oder Lösungen enthalten sein. Diese galenischen Zubereitungen können nach an sich bekannten Methoden hergestellt werden unter Verwendung üblicher fester Trägerstoffe wie z.B. Milchzucker, Stärke oder Talkum oder flüssiger Verdünnungsmittel wie z.B. Wasser, fetten Ölen oder flüssigen Paraffinen und unter Verwendung von pharmazeutisch üblichen Hilfsstoffen, beispielsweise Tablettensprengmitteln, Lösungsvermittlern oder Konservierungsmitteln.

Die nachfolgenden Beispiele sollen die Erfindung näher erläutern.

Beispiel 1:

3-{[4-(2-Pyridyl)-piperazin-1-yl]-carbonyl}-flavon.

a) Zur Herstellung eines gemischten Anhydrides werden 6 g Flavon-3-carbonsäure in 50 ml absolutem Dichlormethan gelöst und unter Feuchtigkeitsausschluß auf -20 °C gekühlt. Zu der gekühlten Mischung gibt man direkt nacheinander 5,5 g Dimethylaminopyridin und 2,2 ml Methansulfonylchlorid.
b) Zu der nach a) hergestellten das gemischte Anhydrid enthaltenden Reaktionslösung werden nach 10 min 3,3 g 1-(2-Pyridyl)-piperazin gegeben. Das Reaktionsgemisch wird 30 min bei -20 °C und noch weitere 2 Stunden bei Raumtemperatur gerührt. Anschließend wird das Reaktionsgemisch zur Aufarbeitung in 100 ml gesättigte Natriumbicarbonatlösung gegossen und ausgeschüttelt. Die organische Phase wird abgetrennt, über Natriumsulfat getrocknet und eingeengt. Es werden 11,9 g öliges Rohprodukt erhalten. Dieses wird mittels Säulenchromatographie an Kieselgel unter leicht erhöhtem Druck (Flashchromatographie) unter Verwendung von Äthylacetat/Methanol 7:3 als Elutionsmittel gereinigt. Es werden 9 g kristallines Rohprodukt erhalten, welches nochmals aus Äther umkristallisiert wird. Hierbei werden 5 g reines 3-{[4-(2-Pyridyl)-piperazin-1-yl]-carbonyl}-flavon erhalten.
Summenformel: $C_{25}H_{21}N_3O_3$, Molekulargewicht: 411,5 Fp: 178-181 °C.
c) Zur Überführung in das Hydrochlorid werden 2,1 g der Titelverbindung in Dichlormethan gelöst und die Lösung wird ca. 5 min mit Chlorwasserstoff-Gas unter Eiskühlung begast. Anschließend wird zur Trockne eingeengt und das verbleibende Kristallisat in Diäthyläther aufgenommen, einige Zeit gerührt und abgesaugt.
Man erhält 2,19 g des Monohydrochlorids der Titelverbindung welches als Dihydrat kristallisiert.
Summenformel: $C_{25}H_{21}N_3O_3.HCl.2H_2O$, Molekulargewicht: 484, Fp: 135-139 °C.

Beispiel 2:

3-[(4-Benzyl-piperazin-1-yl)-carbonyl]-flavon.

a) 6 g Flavon-3-carbonsäure werden mit 5,5 g 4-Dimethylaminopyridin und 2,2 ml Methansulfonylchlorid in 50 ml absolutem Dichlormethan unter den in Beispiel 1 a) beschriebenen Bedingungen in das gemischte Anhydrid überführt und dieses wird anschließend mit 4,42 ml N-Benzyl-piperazin wie in Beispiel 1 b) beschrieben umgesetzt. Die Aufarbeitung des Reaktionsgemisches und die Reinigung der erhaltenen rohen Titelverbindung erfolgen wie in Beispiel 1 b) beschrieben. Es werden 9 g 3-[(4-Benzyl-piperazin-1-yl)-carbonyl]-flavon erhalten.
Summenformel: $C_{27}H_{24}N_2O_3$, Molekulargewicht: 424,5 Fp.: 149-150 °C.
b) Zur Überführung in ihr Monotartrat werden 2 g der Titelverbindung in Äthanol gelöst und mit einer Lösung von 0,27 g (=1 Äquivalent) L(+)-Weinsäure in Äthanol versetzt. Die Mischung wird eingeengt, wobei das 3-[(4-Benzyl-piperazin-1-yl)-carbonyl]-flavon-tartrat-monohydrat auskristallisiert. Es werden 2,4 g des reinen Tartrats erhalten.
Summenformel: $C_{31}H_{30}N_2O_9.1H_2O$, Molekulargewicht: 592,6 Fp: 117-120 °C.

Beispiel 3

3-[3-(Diäthylamino)-propoxycarbonyl]-flavon.

a) 4,0 g Flavon-3-carbonsäure werden zur Herstellung eines gemischten Anhydrides in 100 ml Dichlormethan gelöst, mit 3,67 g Dimethylaminopyridin versetzt und auf -20 °C abgekühlt. Zu der gekühlten Lösung werden 1,28 ml Methansulfonsäurechlorid zugegeben und das Reaktionsgemisch 10 Minuten gerührt.
b) Zu dem nach a) hergestellten das gemischte Anhydrid enthaltenden gekühlten Reaktionsgemisch werden 1,28 ml 3-Diäthylaminopropan-1-ol zugegeben, und das Reaktionsgemisch wird 2 Stunden bei Raumtemperatur reagieren gelassen. Zur Aufarbeitung wird das Reaktionsgemisch anschließend mit gesättigter Natriumbicarbonatlösung geschüttelt, die organische Phase wird abgetrennt, mit Wasser gewaschen, über Natriumsulfat getrocknet und eingeengt. Das noch mit Dimethylaminopyridin verunreinigte Rohprodukt wird mittels Säulenchromatographie an Kieselgel unter leicht erhöhtem Druck (Flashchromatographie) unter Verwendung von Methyläthylketon/Diäthylamin 30:1 als Elutionsmittel

gereinigt. Es werden 4,94 g öliges Rohprodukt erhalten. Dieses wird zur weiteren Reinigung in Äther gelöst, die Ätherlösung mit 1n wäßriger Salzsäurelösung ausgeschüttelt, die wäßrige Phase abgetrennt, mit Natriumbicarbonatlösung neutralisiert und mit Äther ausgeschüttelt. Die Ätherphase wird nochmals mit Wasser gewaschen, über Natriumsulfat getrocknet und eingeengt. Es werden 4,0 g der Titelverbindung als Öl erhalten.
Zur Überführung in das Hydrochlorid werden 3,7 g der vorstehend erhaltenen Titelverbindung in absolutem Dichlormethan gelöst und die Lösung mit Chlorwasserstoffgas begast. Anschließend wird eingeengt, wobei das Hydrochlorid der Titelverbindung auskristallisiert. Es werden 3,8 g 3-[3-(Diäthylamino)-propoxycarbonyl]-flavon-hydrochlorid erhalten.
Summenformel: $C_{23}H_{26}NO_4Cl$, Molekulargewicht: 415.92, Fp: 173-179 °C.

Beispiel 4:

3-{4-[4-(2-Pyridyl)-piperazin-1-yl]-butylaminocarbonyl}-flavon.

a) 28,1 ml 1-(2-Pyridyl)-piperazin und 55,3 ml 1,4-Dibrombutan werden in 150 ml Isopropanol gelöst und in der Lösung werden 16,6 g Natriumcarbonat suspendiert. Anschließend wird filtriert, das Filtrat am Rotationsverdampfer um 1/3 eingeengt und 12 Stunden im Kühlschrank gekühlt. Sodann wird das auskristallisierte Spirobutylpiperazinium-bromid abgesaugt, mit Äther gewaschen und getrocknet. Es werden 52,9 g des Spirobutylpiperaziniumbromid erhalten.
b) 30 g des vorstehend erhaltenen Spirobutylpiperaziniumbromid werden in 400 ml absolutem Dimethylformamid gelöst und mit 13,3 g Natriumazid versetzt. Das Reaktionsgemisch wird 8 Stunden unter Rückfluß erhitzt. Nach dem Abkühlen wird das Gemisch mit dem gleichen Volumen Diäthyläther versetzt und mit gesättigter Natriumcarbonatlösung ausgeschüttelt. Die Ätherphase wird abgetrennt, die wäßrige Natriumbicarbonatlösung noch zweimal nachextrahiert und die vereinigten Ätherphasen werden über Natriumsulfat getrocknet und eingeent. Es werden 17,3 g 4-(4-Azidobutyl)-1-(2-pyridyl)-piperazin als braunrotes Öl erhalten. Das Produkt wird ohne Reinigung weiterverarbeitet.
c) 17,3 g der vorstehend erhaltenen Azid-Verbindung werden in 300 ml Methanol gelöst. Die Lösung wird mit 10 g Raney-Nickel versetzt, und das Azid wird bei 3 bar Wasserstoffdruck zum entsprechenden Amin hydriert. Nach vollendeter Hydrierung wird das Raney-Nickel abgesaugt, mit Methanol gewaschen und die vereinigten Methanol-Phasen werden eingeengt. Es verbleiben 14,2 g eines öligen Rohproduktes, welches teilweise kristallisiert. Nach Abtrennen der Kristalle wird der verbleibende Teil des Rohproduktes mittels Säulenchromatographie an Kieselgel unter leicht erhöhtem Druck unter Verwendung von Methanol/Triäthylamin 30:2 gereinigt. Es werden 12,9 g 4-[4-(2-Pyridyl)-piperazin-1-yl]-butylamin erhalten, Fp: 161-168 °C.
d) Zu einem auf -20 °C abgekühlten Reaktionsgemisch, welches aus 4,0 g Flavon-3-carbonsäure, 3,367 g Dimethylaminopyridin und 1,28 ml Methansulfonsäurechlorid analog Beispiel 1a hergestelltes gemischtes Anhydrid in 100 ml absolutem Dichlormethan enthält, werden 3,6 g des vorstehend erhaltenen 4-[4-(2-Pyridyl)-piperazin-1-yl]-butylamin gegeben. Das Reaktionsgemisch wird sich auf Raumtemperatur erwärmen gelassen und 2 Stunden bei Raumtemperatur gerührt. Anschließend wird das Reaktionsgemisch analog Beispiel 3 aufgearbeitet und die erhaltene wird Titelverbindung aus Äthylacetat umkristallisiert. Man erhält 4,7g 3-{4-[4-(2-Pyridyl)-piperazin-1-yl]-butylaminocarbonyl}-flavon.
Summenformel: $C_{29}H_{30}N_4O_3$, Molekulargewicht: 482,59, Fp: 170-176 °C.
e) Die Titelverbindung wird unter den in Beispiel 1c beschriebenen Bedingungen mit Chlorwasserstoff-Gas umgesetzt. Man erhält das amorphe Trihydrochlorid der Titelverbindung.
Summenformel: $C_{29}H_{33}N_4O_3Cl_3$ . 3,34 $H_2O$, Molekulargewicht: 652,17

Beispiel 5:

3-[(4-Benzylpiperazin-1-yl)-carbonyl]-flavon-N-oxid.

3-[(4-Benzylpiperazin-1-yl)-carbonyl]-flavon (Herstellung siehe Beispiel 2) werden in 50 ml Dichlormethan gelöst und mit 1,4 g m-Chlorperbenzoesäure versetzt. Das Reaktionsgemisch wird 1 Stunde bei Raumtemperatur gerührt, wobei die N-Oxidation vollständig abläuft. Zur Aufarbeitung wird das Reaktionsgemisch anschließend eingeengt, der Rückstand in Methanol gelöst und mit zwei Löffeln eines stark basischen Anionenaustauschers (Handelsprodukt Merck III, Hersteller Fa. Merck oder Handelsprodukt Amberlite ® IRA 400, Hersteller Fa. Rohm und Haas) versetzt und ca. 2 Stunden gerührt. Anschließend wird filtriert, das Filtrat eingeengt und das verbleibende Rohprodukt in Äther aufgenommen. Nach einiger Zeit

kristallisiert das Produkt aus. Man erhält 2,95 g der Titelverbindung.
Summenformel: $C_{27}H_{24}N_2O_4.2,1\ H_2O$, Molekulargewicht: 440,5, Fp: 194-196 °C.

Beispiel 6:

3′,4′,5′-Trimethoxy-3-{4-[4-(2-pyridyl)-piperazin-1-yl]-butylaminocarbonyl}-flavon.

a) 32,6 g 3,4,5-Trimethoxybenzoesäurechlorid werden in 50 ml Pyridin gelöst, und zu der Lösung werden unter Rühren 17 ml 2-Hydroxyacetophenon gegeben und das Reaktionsgemisch 1 Stunde auf 80 °C erwärmt. Nach dem Abkühlen wird das Gemisch zur Aufarbeitung auf eine Mischung aus 45 ml 37%-iger Salzsäure, 45 ml Wasser und 500 g Eis gegossen. Das ausfallende Produkt wird abfiltriert, mit Wasser neutral gewaschen und getrocknet. Es werden 44,7 g 3,4,5-Trimethoxybenzoesäure-(2′-methylcarbonylphenyl)-ester erhalten, welcher ohne Reinigung weiterverarbeitet wird.
b) Für die Baker-Venkatamaran-Umlagerung wird eine Lösung des unter a) erhaltenen Benzoesäureesters in 80 ml Pyridin unter Eiskühlung zu einer Suspension von 42,5 g fein zerstoßenem Natriumhydroxid in 50 ml absolutem Pyridin gegeben. Das Kühlbad wird entfern und das Reaktionsgemisch kräftig durchgerührt. Nach 2 Stunden wird das Reaktionsgemisch zur Aufarbeitung in eine Mischung aus 350 ml konzentrierter Salzsäure und 750 ml zerstoßenem Eis gegossen. Das gelbgefärbte Produkt fällt aus und wird abgesaugt, mit Wasser neutral gewaschen und getrocknet. Es werden 42,6 g 2-Hydroxy-3′,4′,5′-trimethoxybenzoylacetophenon erhalten, welches sofort weiterverarbeitet wird.
c) 42,6 g des vorstehend erhaltenen Diketons werden mit 150 ml Eisessig und 5,7 ml konzentrierter Schwefelsäure versetzt und das Reaktionsgemisch wird während 1 1/2 Stunden auf 120 °C erwärmt. Nach dem Abkühlen wird das Gemisch zur Aufarbeitung auf 500 g Eis gegossen und die Mischung kräftig gerührt, wobei das gebildete 3′,4′,5′-Trimethoxy-flavon kristallisiert. Es wird abfiltriert, mit Wasser gewaschen und getrocknet. Man erhält 38,9 g 3′,4′,5′-Trimethoxyflavon, welches direkt weiterverarbeitet werden kann.
d) Unter Stickstoffatmosphäre werden 9,9 ml Diisopropylamin mit 12 ml Tetrahydrofuran versetzt, und zu dieser Mischung werden bei 0 °C 44,5 ml 1,6 n Butyllithium/-n-Hexan hinzugefügt und die Mischung auf -60 °C gekühlt. Dann werden weitere 50 ml absolutes Tetrahydrofuran zugefügt und die Lösung auf -78 °C gekühlt. Eine auf ca. -60 °C gekühlte Lösung von 15 g 3′,4′,5′-Trimethoxyflavon in 500 ml absolutem Tetrahydrofuran werden dann schnell zu der Lithiiumdiisopropylamid-Lösung gegeben. Nach 3 min werden 10 g frisch zerstoßenes Trockeneis ($CO_2$, -78 °C) zugefügt. Sodann wird die Kühlung entfernt und das Gemisch sich auf Raumtemperatur erwärmen lassen, wobei überschüssiges Kohlendioxid gasförmig aus der Lösung gerührt wird. Zur Aufarbeitung wird das Reaktionsgemisch mit halbkonzentrierter Salzsäure auf pH1 gebracht und mit Dichlormethan extrahiert. Die Dichlormethanphase wird mit einer wäßrigen gesättigten Natriumbicarbonatlösung (ca. 100 ml) ausgeschüttelt. Die wäßrige Phase wird abgetrennt und vorsichtig mit konzentrierter Salzsäure auf pH1 gebracht, wobei die gebildete 3′,4′,5′-Trimethoxyflavon-3-carbonsäure ausfällt. Das ausgefallene Produkt wird mit Dichlormethan aus dem Reaktionsgemisch extrahiert. Die Dichlormethanphase wird abgetrennt, über Natriumsulfat getrocknet und eingeengt, wobei das Produkt kristallisiert. Es werden 14,75 g 3′,4′,5′-Trimethoxy-flavon-3-carbonsäure erhalten.
Summenformel: $C_{19}H_{16}O_7$, Molekulargewicht: 356,34 Fp: 260-262 °C.
e) 3,0 g 3′,4′,5′-Trimethoxyflavon-3-carbonsäure werden mit 2,1 g 4-Dimethylaminopyridin und 0,72 ml Mesylchlorid in 100 ml absolutem Dichlormethan unter den in Beispiel 1 a) beschriebenen Bedingungen in das gemischte Anhydrid überführt und dieses wird anschließend unter den in Beispiel 4 d) beschriebenen Bedingungen mit 2,2 g 4-[4-(2-pyridyl)-piperazin-1-yl]-butylamin umgesetzt. Das Reaktionsgemisch wird anschließend analog Beispiel 4 d) aufgearbeitet. Es werden 3,4 g 3′,4′,5′-Trimethoxy-3-{4-[4-(2-pyridyl)-piperazin-1-yl]-butylaminocarbonyl}-flavon erhalten. Summenformel: $C_{32}H_{36}N_4O_6$, Molekulargewicht: 572,67, Fp.: 168-173 °C.
f) Die Titelverbindung wird analog Beispiel 1c mit Chlorwasserstoff-Gas umgesetzt. Es wird das amorphe Monohydrochlorid der Titelverbindung erhalten.
Summenformel: $C_{32}H_{36}N_4O_6\ .\ 2,2\ HCl\ .\ 3\ H_2O$, Molekulargewicht: 706,93

Beispiel 7:

3',4',5'-Trimethoxy-3-{[4-(2-pyridyl)-piperazin-1-yl]-carbonyl}-flavon.

a) 8 g 3',4',5'-Trimethoxy-flavon-3-carbonsäure werden mit 5,5 g 4-Dimethylaminopyridin und 2,2 ml Mesylchlorid in 100 ml absolutem Dichlormethan analog Beispiel 1 a) in das gemischte Anhydrid überführt und dieses wird anschließend analog Beispiel 1 b) mit 3,6 g 1-(2-Pyridyl)-piperazin umgesetzt. Die Aufarbeitung des Reaktionsgemisches und die Reinigung der erhaltenen rohen Titelverbindung werden analog Beispiel 1 b) durchgeführt. Man erhält 7,5 g 3',4',5'-Trimethoxy-3-{[4-(2-pyridyl)-piperazin-1-yl]-carbonyl}-flavon.
Summenformel: $C_{28}H_{27}N_3O_6$.0,5$H_2O$, Molekulargewicht: 510,55 Fp.: 90-95 °C.
b) Die Titelverbindung wird analog Beispiel 1c mit Chlorwasserstoff-Gas umgesetzt. Es wird das Monohydrochlorid erhalten.
Summenformel: $C_{28}H_{28}N_3O_6Cl$ . 1,5 $H_2O$, Molekulargewicht: 565,02 Fp.: 135-140

Beispiel 8:

3',4',5'-Trimethoxy-3-[(4-benzylpiperazin-1-yl)-carbonyl]-flavon.

a) 6 g 3',4',5'-Trimethoxy-flavon-3-carbonsäure werden mit 4,1 g 4-Dimethylaminopyridin und 1,6 ml Mesylchlorid in absolutem Dichlormethan unter den in Beispiel 1a) angegebenen Bedingungen in das gemischte Anhydrid überführt. Dieses wird analog Beispiel 1 b) mit 3,3 ml 1-Benzylpiperazin umgesetzt. Das Reaktionsgemisch wird analog Beispiel 1 b) aufgearbeitet. Man erhält 7,0 g des reinen 3',4',5'-Trimethoxy-3-[(4-benzylpiperazin-1-yl)-carbonyl]-flavon.
Summenformel: $C_{30}H_{30}N_2O_6$.0,5$H_2O$, Molekulargewicht: 523,58, Fp.: 195-198 °C.
b) Die Titelverbindung wird analog Beispiel 1c mit Chlorwasserstoff-Gas umgesetzt. Es wird das Monohydrochlorid erhalten.
Summenformel: $C_{30}H_{31}N_2O_6Cl$ . 1,5 $H_2O$, Molekulargewicht: 578,06 Fp.: 210-214 °C

Beispiel 9:

3',4',5'-Trihydroxy-3-{4-[4-(2-pyridyl)-piperazin-1-yl]-butylaminocarbonyl}-flavon.

a) 3,5 g 3',4',5'-Trimethoxy-3-{4-[4-(2-pyridyl)-piperazin-1-yl]-butylaminocarbonyl}-flavon (hergestellt nach Beispiel 7) werden in 200 ml Dichlormethan gelöst. Die Lösung wird unter Stickstoffatmosphäre auf 0 °C abgekühlt. Zu der abgekühlten Lösung werden 43 ml einer 1-molaren Bortribromid-Lösung in Dichlormethan gegeben. Das Reaktionsgemisch wird 12 Stunden lang gerührt, wobei es sich auf Raumtemperatur erwärmen kann. Anschließend wird das Reaktionsgemisch zur Aufarbeitung vorsichtig mit Wasser versetzt und mit gesättigter Natriumbicarbonatlösung auf pH7 neutralisiert. Hierbei fällt die Titelverbindung in verunreinigter Form aus und wird abfiltriert und bei 80 °C getrocknet.
b) Zur weiteren Reinigung wird die Trihydroxy-Verbindung in ihr Triacetat überführt. Hierzu werden 3 g der rohen Titelverbindung in einer Mischung aus 32 ml Essigsäureanhydrid, 32 ml Pyridin und 50 ml Dichlormethan suspendiert und das Gemisch solange gerührt, bis eine fast homogene Lösung vorliegt. Dann wird das Reaktionsgemisch mit Eiswasser ausgeschüttelt und die organische Phase wird abgetrennt und über Natriumsulfat getrocknet. Um Reste von Pyridin abzutrennen wird 2 mal unter Zusatz von Toluol eingeengt. Der verbleibende Rückstand wird mittels Säulenchromatographie an Kieselgel unter leicht erhöhtem Druck (Flash-Chromatographie) unter Verwendung von Tetrahydrofuran/Dichlormethan 3:1 als Elutionsmittel gereinigt. Hierbei wird reines 3',4',5'-Triacetoxy-3-{4-[4-(2-pyridyl)-piperazin-1-yl]-butylaminocarbonyl}-flavon erhalten.
c) Zur Herstellung der reinen Trihydroxy-Titelverbindung wird die vorstehend erhaltene saubere Triacetoxy-Verbindung in absolutem Methanol gelöst und die Lösung mit 3 Tropfen Triäthylamin versetzt. Unter diesen Bedingungen findet eine Umesterung statt, deren Verlauf dünnschichtchromatographisch kontrolliert wird. Sobald keine Triacetoxy-Verbindung mehr dünnschichtchromatographisch nachgewiesen werden kann und die Umesterung somit vollständig ist, wird das Reaktionsgemisch eingeengt, der Rückstand mit Wasser aufgerührt, abfiltriert und nochmals mit Wasser gewaschen. Man erhält 1,0 g der amorphen reinen Titelverbindung.
Summenformel: $C_{29}H_{30}N_4O_6$, Molekulargewicht: 530,59

Beispiel 10:

3′,4′,5′-Trihydroxy-3-{[4-(2-pyridyl)-piperazin-1-yl]-carbonyl -flavon.

a) 13,4 g 3′,4′,5′-Trimethoxy-3-{[4-(2-pyridyl)-piperazin-1-yl]-carbonyl}-flavon (hergestellt analog Beispiel 6) werden mit 107,2 ml einer 1-molaren Bortribromidlösung in Dichlormethan wie in Beispiel 9 a) beschrieben umgesetzt. Das Reaktionsgemisch wird wie in Beispiel 9 a) beschrieben aufgearbeitet, wobei 9 g getrocknetes Rohprodukt erhalten wird. Dieses wird wie in Beispiel 9 b) beschrieben mit einem Gemisch aus 50 ml Pyridin und 50 ml Acetanhydrid in das Triacetat der Titelverbindung überführt und dieses wie in Beispiel 9 b) gereinigt und anschließend analog Beispiel 9 c) in die reine Titelverbindung überführt. Es werden 4 g reines amorphes 3′,4′,5′-Trihydroxy-3-{[4-(2-Pyridyl)-piperazin-1-yl]-carbonyl}-flavon erhalten.
Summenformel: $C_{25}H_{21}N_3O_6$, Molekulargewicht: 459,46.
b) Die Titelverbindung wird analog Beispiel 1c mit Chlorwasserstoff-Gas umgesetzt. Es wird das Monohydrochlorid erhalten.
Summenformel: $C_{25}H_{22}N_3O_6Cl$ . 0,7 $H_2O$, Molekulargewicht: 508,79 Fp.: 244-250 °C

Beispiel 11:

3′,4′,5′-Tripivaloyloxy-3-{[4-(2-pyridyl)-piperazin-1-yl]-carbonyl}-flavon.

4,0 g 3′,4′,5′-Trihydroxy-3-{[4-(2-pyridyl)-piperazin-1-yl]-carbonyl}-flavon werden in 50 ml absolutem Pyridin gelöst und die Lösung mit 3,2 ml Pivalonsäurechlorid versetzt. Das Reaktionsgemisch wird 12 Stunden bei Raumtemperatur stehengelassen. Anschließend wird zur Aufarbeitung auf 150 ml eines Eis/Wasser-Gemisches gegossen und mit Methylenchlorid extrahiert. Die Methylenchloridphase wird abgetrennt, über Natriumsulfat getrocknet, eingeengt und noch 3 mal mit Toluol versetzt und wiederum eingeengt. Der verbleibende Rückstand wird mittels Säulenchromatographie an Kieselgel unter leicht erhöhtem Druck unter Verwendung von Äthylacetat/Dichlormethan 8:2 als Elutionsmittel gereinigt. Man erhält 2,3 g kristallines 3′,4′,5′-Tripivaloyloxy-3-{[4-(2-pyridyl)-piperazin-1-yl]-carbonyl}-flavon, das mit 1/2 Mol Wasser kristallisiert.
Summenformel: $C_{40}H_{45}N_3O_9$, Molekulargewicht: 720,82 Fp: 120-125 °C.

Beispiel 12:

3′,4′,5′-Trimethoxy-3-{2-[4-(2-pyridyl)-piperazin-1-yl]-äthylaminocarbonyl}-flavon

a) Zur Überführung in ein gemischtes Anhydrid werden 7,13 g 3′,4′,5′-Trimethoxyflavon-3-carbonsäure in 100 ml absolutem Dichlormethan gelöst, auf -20 °C gekühlt und unter Feuchtigkeitsausschluß mit 4,9 g Dimethylaminopyridin und 1,9 ml Mesylchlorid versetzt. Das Reaktionsgemisch wird 10 min bei -20 °C reagieren gelassen.
b) Zu der vorstehend hergestellten, das gemischte Anhydrid der 3′,4′,5′-Trimethoxyflavon-3-carbonsäure enthaltenden Reaktionslösung werden 1,33 ml 2-Aminoäthanol zugegeben. Das Reaktionsgemisch wird sich auf Raumtemperatur erwärmen lassen und noch einige Zeit gerührt. Anschließend wird das Gemisch zur Aufarbeitung in gesättigte Natriumbicarbonatlösung gegeben, die organische Phase abgetrennt und die wäßrige Phase nochmals mit Dichlormethan extrahiert. Die vereinigten Dichlormethanphasen werden über Natriumsulfat getrocknet und eingeengt. Das hierbei teilweise auskristallisierende 3′,4′,5′-Trimethoxy-3-(2-hydroxyäthylaminocarbonyl)-flavon wird abgetrennt. Aus der Mutterlauge kristallisiert nach weiterem Einengen nochmals ein Teil des Produktes aus. Man erhält insgesamt 6,2 g.
c) 0,4 g 3′,4′,5′-Trimethoxy-3-(2-hydroxyäthylaminocarbonyl)-flavon werden in 20 ml Dichlormethan gelöst. Die Lösung wird mit 0,2 ml Phosphortribromid versetzt, kurz auf 60 °C erwärmt und sodann noch 12 Stunden bei Raumtemperatur stehengelassen. Zur Aufarbeitung wird das Reaktionsgemisch auf Eiswasser gegossen, die organische Phase abgetrennt und noch 3 mal mit gesättigter Natriumbicaronatlösung gewaschen, über Natriumsulfat getrocknet und eingeengt. Der verbleibende Rückstand wird in Äther aufgenommen. Aus der Äther-Lösung kristallisieren 0,3 g des 3′,4′,5′-Trimethoxy-3-(2-bromoäthylaminocarbonyl)-flavons. Dieses wird direkt weiterverarbeitet.
d) 0,2 g 3′,4′,5′-Trimethoxy-3-(2-bromoäthylaminocarbonyl)-flavon werden in 10 ml Dichlormethan gelöst und die Lösung wird mit einer Spatelspitze Kaliumjodid und mit 0,5 ml 1-(2-Pyridyl)-piperazin versetzt. Das Reaktionsgemisch wird 1 Stunde auf Rückflußtemperatur erhitzt. Nach dem Abkühlen wird zur

Aufarbeitung weiteres Dichlormethan zugesetzt und mit gesättigter Natriumbicarbonatlösung und Wasser ausgeschüttelt. Sodann wird die organische Phase über Natriumsulfat getrocknet, eingeengt und der verbleibende Rückstand mittels Säulenchromatographie an Kieselgel unter leicht erhöhtem Durck (Flash-Chromatographie) unter Verwendung von Tetrahydrofuran/Äthylacetat 1:1 als Elutionsmittel gereinigt. Hierbei wird das 3′,4′,5′-Trimethoxy-3-{2-[4-(2-pyridyl)-piperazin-1-yl]-äthylaminocarbonyl}-flavon in reiner amorpher Form erhalten.
Summenformel: $C_{30}H_{32}N_4O_6$, Molekulargewicht: 546,36
e) Die Titelverbindung wird analog Beispiel 1c mit Chlorwasserstoff-Gas umgesetzt. Es wird das amorphe Hydrochlorid der Titelverbindung erhalten.
Summenformel: $C_{30}H_{32}N_4O_6$.2,7 HCl.3,9 $H_2O$, Molekulargewicht: 713,32

Beispiel 13:

3′,4′,5′-Trimethoxy-3-(piperazin-1-yl-carbonyl)-flavon.

0,4 g 3′,4′,5′-Trimethoxy-3-[(4-benzylpiperazin-1-yl)-carbonyl]-flavon werden in 10 ml Äthanol gelöst. Zu der Lösung werden 1 ml konzentrierte Salzsäure und Palladium/Kohle (5 %) als Katalysator zugegeben. Sodann wird bei einem Wasserstoffdruck von 5 bar 5 Stunden lang hydriert. Anschließend wird der Katalysator abfiltriert und das Filtrat eingeengt. Es werden 210 mg des Hydrochlorids der amorphen Titelverbindung erhalten.
Summenformel: $C_{23}H_{24}N_2O_6$.1HCl, Molekulargewicht: 460,70

Beispiel 14:

3′,4′,5′-Trihydroxy-3-[(4-benzylpiperazin-1-yl)-carbonyl]-flavon.

a) 4,0 g 3′,4′,5′-Trimethoxyflavon-3-carbonsäure werden in 200 ml absolutes Dichlormethan gegeben und mit 82 ml einer 1-molaren Bortribromid-Lösung in absolutem Dichlormethan unter Stickstoffatmosphäre bei 0 °C versetzt. Das Reaktionsgemisch wird 12 Stunden gerührt, wobei es sich auf Raumtemperatur erwärmen kann. Anschließend wird zur Aufarbeitung unter Eiskühlung vorsichtig Wasser zugesetzt und dann mit Wasser auf das doppelte Volumen aufgefüllt und intensiv gerührt. Hierbei fällt die gebildete 3′,4′,5′-Trihydroxyflavon-3-carbonsäure aus und wird abfiltriert. Der Filterkuchen wird mehrfach mit Wasser gewaschen und aus Methanol umkristallisiert. Es werden 3 g reiner 3′,4′,5′-Trihydroxyflavon-3-carbonsäure erhalten.
Summenformel: $C_{16}H_{10}O_7$.1$H_2O$, Molekulargewicht: 332,27, Fp: 230 °C.
b) 3′,4′,5′-Trihydroxyflavon-3-carbonsäure werden in 4 ml absolutem Pyridin gelöst und die Lösung mit 0,57 ml Acetanhydrid versetzt und zwei Stunden gerührt. Sodann wird zur Aufarbeitung mit 20 ml mit Dichlormethan verdünnt, auf eine Mischung aus verdünnter Salzsäure/Eiswasser gegossen und mit Dichlormethan ausgeschüttelt. Hierbei muß dafür gesorgt werden, daß die wäßrige Phase sauer bleibt (gegebenenfalls muß etwas verdünnte Salzsäure zugesetzt werden). Die Dichlormethan-Phase wird abgetrennt, mit Wasser gewaschen, über Natriumsulfat getrocknet und eingedampft. Man erhält 0,8 g rohe 3′,4′,5′-Triacetoxyflavon-3-carbonsäure, welche ohne weitere Reinigung sofort weiterverarbeitet wird.
c) Die vorstehend erhaltene rohe 3′,4′,5′-Triacetoxyflavon-3-carbonsäure wird in 10 ml Dichlormethan gelöst und zur Überführung in ihr gemischtes Anhydrid unter Feuchtigkeitsausschluß bei -20 ~C mit 0,44 g Dimethylaminopyridin und 0,16 ml Mesylchlorid versetzt. Das Reaktionsgemisch wird solange reagieren gelassen, bis dünnschichtchromatographisch keine freie Säure mehr nachzuweisen ist.
d) Zu der vorstehend hergestellten, das gemischte Anhydrid enthaltenden Reaktionslösung wird ein Überschuß von 4-Benzylpiperazin (1 ml) hinzugefügt. Das Reaktionsgemisch wird zur Bilding der Titelverbindung noch 10 min bei -20 °C und 1 Stunde bei Raumtemperatur gerührt. Anschließend wird das Reaktionsgemisch zur Aufarbeitung eingeengt, der verbleibende Rückstand in Methanol aufgenommen, die methanolische Lösung einige Zeit stehengelassen, um eine vollständige Deacylierung zu gewährleisten, sodann eingeengt, der Rückstand in Wasser aufgenommen und mit Äthylace tat extrahiert. Die organische Phase wird abgetrennt, über Natriumsulfat getrocknet und eingeengt. Die verbleibende rohe Titelverbindung wird mittels Säulenchromatographie an Kieselgel unter leicht erhöhtem Druck (Flash-Chromatographie) unter Verwendung von Dichlormethan/Methanol 1:1 als Elutionsmittel gereinigt. Es werden 200 mg 3′,4′,5′-Trihydroxy-3[(4-benzylpiperazin-1-yl)-carbonyl]-flavon erhalten.
Summenformel: $C_{27}H_{24}N_2O_6$.1,2$H_2O$, Molekulargewicht: 494,12, Fp: 168-170 °C.

e) Die Titelverbindung wird analog Beispiel 2b in ihr Monotartrat überführt, wobei das 3′,4′,5′-Trihydroxy-3-[(4-benzylpiperazin-1-yl)-carbonyl]-flavon-tartrat-dihydrat auskristallisiert.
Summenformel: $C_{31}H_{30}N_2O_{12}$ . 2 $H_2O$, Molekulargewicht: 658,60 Fp.: 160-165 °C

Nach den in den vorstehenden Beispielen beschriebenen Verfahren können auch die in der folgenden Tabelle 1 aufgeführten Verbindungen der Formel I hergestellt werden.

Nach den in Beipiel 6a-6d beschriebenen Vefahren können die in der nachfolgenden Tabelle 2 aufgeführten Flavon-3-carbonsäuren der Formel II und deren Salze hergestellt werden

TABELLE 1

| Bsp Nr. | $R^1$ | $R^2$ | $R^3$ | $R^4$ | $R^5$ | $R^6$-Z- | Bemerkungen S=Summenformel MG=Molekulargewicht Fp. in °C |
|---|---|---|---|---|---|---|---|
| 15 | 4'-O-$(CH_2)_{19}$-$CH_3$ | H | H | H | H | 4-(2-pyrid)-pip- | HCl:S=$C_{45}H_{61}N_3O_4$ .2HCl MG=780,92 am |
| 16 | H | H | H | H | H | 4-$CH_3$-pip- | a) B: S=$C_{21}H_{20}N_2O_3$ MG=348,4 Fp:187-190<br>b) Tart.0,25$H_2O$ S=$C_{25}H_{26}N_2O_9$ .0,25$H_2O$ MG=503 Fp:203-205 |
| 17 | H | H | H | H | H | 4-(2-pyrim)-pip- | B: S=$C_{24}H_{20}N_4O_3$ MG=412,44 Fp:191 |
| 18 | H | H | H | H | H | 1-benz-pipe-NH- | a) B: S=$C_{28}H_{26}N_2O_3$ MG=438,52 Fp: 183-185<br>b) Tart.1,1$H_2O$ S=$C_{32}H_{32}N_2O_9$ .1,1$H_2O$ MG=608,43 Fp:123-126 |
| 19 | H | H | H | H | H | $(C_2H_5)_2N$-$(CH_2)_3$-NH- | B: S= $C_{23}H_{26}N_2O_3$ .0,3$H_2O$ MG=383,88 Fp:143-145 |

| | | | |
|---|---|---|---|
| B = Base | Tart= Tartrat | HCl = Hydrochlorid | am = amorph |
| Z = unter Zersetzung | pip = Piperazin-1-yl | pyrim= 1,3-Pyrimidyl | pyrid= Pyridyl |
| benz = Benzyl | pipe= Piperidin-4-yl | phen = Phenyl | pyrro= Pyrrolidin-1-yl |
| morph= Morpholin-1-yl | | | |

| Bsp Nr. | $R^1$ | $R^2$ | $R^3$ | $R^4$ | $R^5$ | $R^6$-Z- | Bemerkungen<br>S=Summenformel<br>MG=Molekulargewicht<br>Fp. in °C |
|---|---|---|---|---|---|---|---|
| 20 | H | H | H | H | H | pyrro-$(CH_2)_2$-NH- | a) B: S=$C_{22}H_{22}N_2O_3$ . 0,1$H_2O$<br>MG=364,23<br>Fp:137-138<br>b) HCl:S=$C_{22}H_{23}N_2O_3Cl$ .2,2$H_2O$<br>MG 438,52 am |
| 21 | H | H | H | H | H | morph-$(CH_2)_3$-NH- | a) B: S=$C_{23}H_{24}N_2O_4$<br>MG=392,42<br>Fp:147-149<br>b) HCl:S=$C_{23}H_{25}N_2O_4Cl$ .1,6$H_2O$<br>MG=457,75 am |
| 22 | H | H | H | H | H | $(C_2H_5)_2N-(CH_2)_2$-NH- | a) B:S=$C_{22}H_{24}N_2O_3$<br>MG=364,45<br>Fp:147-148<br>b) HCl:S=$C_{22}H_{24}N_2O_3$ .0,2$H_2O$<br>MG 402,69 am |
| 23 | H | H | H | H | H | 4-benz-pip-$(CH_2)_4$-NH- | HCl:S=$C_{31}H_{33}N_3O_3$ .2,35HCl.3,3$H_2O$<br>MG=640,98 am |
| 24 | H | H | H | H | H | 4-$CH_3$-pip-$(CH_2)_4$-NH- | B:S=$C_{25}H_{29}N_3O_3$<br>MG=419,53;<br>Fp: 133-134 |
| 25 | H | H | H | H | H | 4-(2-pyrim)-pip-$(CH_2)_4$-NH- | HCl:S=$C_{28}H_{29}N_5O_3$ .2,5HCl.3,66$H_2O$<br>MG=640,66;<br>Fp:139-148 |
| 26 | H | H | H | H | H | 4-(2-pyrid)-pip-$(CH_2)_4$-NH- | N,N,N-Trioxid:<br>S=$C_{29}H_{30}N_4O_6$ .3,8$H_2O$<br>MG 599,05;<br>Fp:125(Z) |

| Bsp Nr. | $R^1$ | $R^2$ | $R^3$ | $R^4$ | $R^5$ | $R^6$-Z- | Bemerkungen S=Summenformel MG=Molekulargewicht Fp. in °C |
|---|---|---|---|---|---|---|---|
| 27 | H | H | H | H | H | 4-(4-F-phen)-pip-$(CH_2)_4$-NH- | HCl:S=$C_{30}H_{30}N_3O_3F$ .2HCl.1,5$H_2O$ MG=599,53 am |
| 28 | 4'-Q$CH_3$ | H | H | H | H | 4-$CH_3$-pip- | Tart:S=$C_{26}H_{28}N_2O_{10}$ .1$H_2O$ MG=546,53; Fp:202-209 |
| 29 | 3'-OH | 4'-OH | H | H | H | 4-(2-pyrid)-pip- | B:S=$C_{25}H_{21}N_3O_5$ MG=443,46 Fp:243 |
| 30 | 3'-OH | H | H | H | H | 4-benz-pip- | Tart:S=$C_{31}H_{30}N_2O_{10}$ .2$H_2O$ MG=626,62 am |
| 31 | 3'-O$CH_3$ | H | H | H | H | 4-benz-pip- | HCl:S=$C_{28}H_{27}N_2O_4Cl$ .1,7$H_2O$ MG=521,62 am |
| 32 | 3'4'-di-O$CH_3$ | | H | H | H | 4-(2-pyrid)-pip- | a)B: S=$C_{27}H_{25}N_3O_5$ .0,4$H_2O$ MG=478,72 Fp:94-97 b) HCl:S=$C_{27}H_{26}N_3O_5Cl$ .2,3$H_2O$ MG=549,4 Fp:141-145 |
| 33 | 3',4'-di-O$CH_3$ | | H | H | H | 4-benz-pip- | HCl: S=$C_{29}H_{29}N_2O_5Cl$ .0,2$H_2O$ MG=524,61 Fp:149-153 |
| 34 | H | H | H | 5,7-di-O$CH_3$ | | 4-(2-pyrid)-pip- | HCl:S=$C_{27}H_{25}N_3O_5$ .1,1HCl.2$H_2O$ MG=547,65 am |

| Bsp Nr. | $R^1$ | $R^2$ | $R^3$ | $R^4$ | $R^5$ | $R^6$ -Z- | Bemerkungen<br>S=Summenformel<br>MG=Molekulargewicht<br>Fp. in °C |
|---|---|---|---|---|---|---|---|
| 35 | 2'-Cl | H | H | H | H | 4-(2-pyrid)-pip- | HCl:S=$C_{25}H_{20}N_3O_3Cl$<br>.1,3HCl.1,6$H_2O$<br>MG=522,13<br>Fp:130-140 |
| 36 | 4'-$OC_{16}H_{33}$ | H | H | H | H | 4-benz-pip- | B:S=$C_{43}H_{56}N_2O_4$<br>MG=664,93<br>Fp:76-80 |
| 37 | 4'-$OC_{16}H_{33}$ | H | H | H | H | 4-(2-pyrid)-pip- | B:S=$C_{41}H_{53}N_3O_4$<br>MG=651,89<br>Fp:87-90 |
| 38 | 4'-$OC_{16}H_{33}$ | H | H | H | H | 4-benz-pip-$(CH_2)_4$-NH- | B:S=$C_{47}H_{65}N_3O_4$<br>MG=736,06; Fp=83 |
| 39 | 3',4',5'-tri-O-CO-$CH_3$ | | | H | H | 4-(2-pyrid)-pip- | a) B:S=$C_{31}H_{27}N_3O_9$<br>.1$H_2O$<br>MG=603,59<br>Fp:184-188<br>b) Tart:S=$C_{35}H_{33}N_3O_{15}$<br>.2$H_2O$<br>MG=771,69<br>Fp:115-120 |
| 40 | 3',4',5'-tri-$OCH_3$ | | | H | H | pyrro-$(CH_2)_2$-NH- | a) B:S=$C_{25}H_{28}N_2O_6$<br>.0,5$H_2O$<br>MG=461,52<br>Fp:129-133<br>b) HCl:S=$C_{25}H_{29}N_2O_6Cl$<br>.1,1$H_2O$<br>MG=508,79<br>Fp:105-110 |
| 41 | 3',4',5'-tri-$OCH_3$ | | | H | H | $(C_2H_5)_2N-(CH_2)_3$-NH- | a) B:S=$C_{26}H_{32}N_2O_6$<br>MG=468,55<br>Fp:120-122<br>b) Tart:S=$C_{30}H_{38}N_2O_{12}$<br>.1$H_2O$<br>MG=636,65<br>Fp:82-86 |

| Bsp Nr. | $R^1$ | $R^2$ | $R^3$ | $R^4$ | $R^5$ | $R^6$-Z- | Bemerkungen<br>S=Summenformel<br>MG=Molekulargewicht<br>Fp. in °C |
|---|---|---|---|---|---|---|---|
| 42 | 3',4',5'-tri-O-CO-$CH_3$ | | | H | H | 4-benz-pip- | a) B:S=$C_{33}H_{30}N_2O_9$ .0,3$H_2O$<br>MG=604,01<br>Fp=198-204<br>b) Tart:S=$C_{37}H_{36}N_2O_{15}$ .2$H_2O$<br>MG=784,74<br>Fp=135-142 |
| 43 | 3',4',5'-tri-$OCH_3$ | | | H | H | $(C_2H_5)_2N-(CH_2)_2$-NH- | a) B:S=$C_{25}H_{30}N_2O_6$ .0,1$H_2O$<br>MG=456,32<br>Fp:117-120<br>b) Tart:S:$C_{29}H_{36}N_2O_{12}$ .1$H_2O$<br>MG=622,63<br>Fp=85-89 |
| 44 | 3',4',5'-tri-$OCH_3$ | | | H | H | morph-$(CH_2)_2$-NH- | B:S=$C_{26}H_{30}N_2O_7$ .0,2$H_2O$<br>MG=486,13<br>Fp:106-109 |
| 45 | 3',4',5'-tri-$OCH_3$ | | | H | H | $(C_2H_5)_2N-(CH_2)_3$-O- | HCl:S=$C_{26}H_{32}NO_7Cl$ .1,5$H_2O$<br>MG=533,02 am |
| 46 | 3',4',5'-tri-$OCH_3$ | | | H | H | morph-$(CH_2)_3$-NH- | Tart:S=$C_{30}H_{36}N_2O_{13}$ .1,5$H_2O$<br>MG=659,64<br>Fp=95-104 |
| 47 | 3',4',5'-tri-$OCH_3$ | | | H | H | 4-(2-pyrid)-pip-$(CH_2)_2$-O- | di-HCl:S=$C_{30}H_{33}N_3O_7Cl_2$ .2$H_2O$<br>MG=654,54<br>Fp=160-164 |
| 48 | 3',4',5'-tri-$OCH_3$ | | | H | H | (2-pyrid)-$(CH_2)_2$-NH- | HCl:S=$C_{26}H_{25}N_2O_6Cl$ .1,2$H_2O$<br>MG=518,57<br>Fp:123-127 |

| Bsp Nr. | $R^1$ | $R^2$ | $R^3$ | $R^4$ | $R^5$ | $R^6$-Z- | Bemerkungen S=Summenformel MG=Molekulargewicht Fp. in °C |
|---|---|---|---|---|---|---|---|
| 49 | 3',4',5'-tri-$OCH_3$ | | | H | H | pyrro-$(CH_2)_2$-O- | HCl:S=$C_{25}H_{27}NO_7$ .1,1HCl.1,3$H_2O$ MG=517,02 Fp:105-108 |
| 50 | 3',4',5'-tri-$OCH_3$ | | | H | H | (2-pyrid)-$(CH_2)_2$-O- | HC 1:S=$C_{26}H_{24}NO_7Cl$ .1$H_2O$ MG=515,95 Fp=99-103 |
| 51 | 3',4',5'-tri-$OCH_3$ | | | H | H | 4-benz-pip-$(CH_2)_4$-NH- | di-HCl:S=$C_{34}H_{41}N_3O_6Cl_2$ .2$H_2O$.0,2$CH_2Cl_2$ MG=711,64 am |
| 52 | 3',4',5'-tri-$OCH_3$ | | | H | H | 4-benz-pip-$(CH_2)_4$-NH- | N,N-Dioxid:S=$C_{34}H_{39}N_3O_8$ .3$H_2O$ MG=671,71 am |
| 53 | 3',4',5'-tri-OH | | | H | H | (2-pyrid)-$(CH_2)_2$-NH- | HCl:S=$C_{23}H_{19}N_2O_6Cl$ .1,2$H_2O$ MG=476,48 Fp:135-140 |
| 54 | 3',4',5'-tri-$OCH_3$ | | | H | H | 4-(2-pyrim)-pip-$(CH_2)_4$-NH- | di-HCl:S=$C_{31}H_{35}N_5O_6$ .2,8HCl.4,67$H_2O$ MG=759,88 Fp:130-136 |
| 55 | 3',4',5'-tri-$OCH_3$ | | | H | H | 4-(2-pyrid)-pip-$(CH_2)_4$-NH- | N,N,N-Trioxid: S=$C_{32}H_{36}N_4O_9$ .2,9$H_2O$ MG=672,91 Fp:125(Z) |
| 56 | 3',4',5'-tri-O-CO-$C_2H_5$ | | | H | H | 4-(2-pyrid)-pip- | B:S=$C_{34}H_{33}N_3O_9$ MG=627,65 Fp=139-144 |
| 57 | 3',4',5'-tri-O-CO-$CH(CH_3)_2$ | | | H | H | 4-(2-pyrid)-pip- | B:S=$C_{37}H_{39}N_3O_9$ MG=669,73 Fp:194-196 |

| Bsp Nr. | $R^1$ | $R^2$ | $R^3$ | $R^4$ | $R^5$ | $R^6$-Z- | Bemerkungen S=Summenformel MG=Molekulargewicht Fp. in °C |
|---|---|---|---|---|---|---|---|
| 58 | 3',4',5'-tri-O-CO-$(CH_2)_2CH_3$ | | | H | H | 4-(2-pyrid)-pip- | B:S=$C_{37}H_{39}N_3O_9$ MG=669,73 Fp:136-139 |
| 59 | 3',4',5'-tri-$OCH_3$ | | | H | H | 4-(4-F-benz)-pip-$(CH_2)_4$-NH- | HCl:S=$C_{33}H_{36}N_3O_6F$ .2HCl.7,7$H_2O$ MG=801,31 am |
| 60 | 3'-Cl | H | H | 6-$CH_3$ | H | 4-(2-pyrid)-pip- | a) B:S=$C_{26}H_{22}N_3O_3Cl$ MG=459,93 Fp=194-197<br>b) HCl:S=$C_{26}H_{22}N_3O_3Cl$ .1,2HCl.1,0$H_2O$ MG=521,70 Fp=142-146 |
| 61 | 4'-$OCH_3$ | H | H | 5,7-di-$OCH_3$ | | 4-(2-pyrid)-pip- | HCl:S=$C_{28}H_{27}N_3O_6$ .2HCl MG=574,46 am |
| 62 | 4'-OH | H | H | 5,7-di-OH | | 4-(2-pyrid)-pip- | HCl:S=$C_{25}H_{21}N_3O_6$.2HCl MG:532,37 am |
| 63 | 3',4',5'-tri-$OCH_3$ | | | H | H | 4-$CH_3$-pip-$(CH_2)_4$-NH | HCl:S=$C_{28}H_{35}O_6N_3$ .2,6HCl.3,4$H_2O$ MG=664,13 am |
| 64 | 3',4',5'-tri-$OCH_3$ | | | H | H | 4-(2-$C_2H_5$-phen)-pip<br>$(CH_2)_4$-NH- | B:S=$C_{35}H_{41}N_3O_6$ MG=599,73 Fp:176-177 |
| 65 | 3',4',5'-tri-$OCH_3$ | | | H | H | 4-(2-pyrid)-pip-$(CH_2)_3$-NH- | HCl:S=$C_{31}H_{34}O_6N_4$ .2,1HCl.3,2$H_2O$ MG=692,82 am |
| 66 | 3',4',5'-tri-$OCH_3$ | | | H | H | pip-$(CH_2)_4$-NH- | HCl:S=$C_{27}H_{33}N_3O_6$.2HCl MG=568,94 am |

TABELLE 2

| Bsp Nr. | $R^1$ | $R^2$ | $R^3$ | $R^4$ | $R^5$ | Bemerkungen<br>S=Summenformel<br>MG=Molekulargewicht<br>Fp. in °C |
|---|---|---|---|---|---|---|
| 101 | H | H | H | H | H | a) f. Säu: $S=C_{16}H_{10}O_4$<br>MG=266,25 Fp:178-180<br>b) Na-Salz: $S=C_{16}H_9O_4Na$<br>MG 288,23 Fp:314-317 |
| 102 | 3'-$OCH_3$ | H | H | H | H | a) f. Säu: $S=C_{17}H_{12}O_5$<br>MG=296,28 Fp:171-173<br>b) Na-Salz: $S=C_{17}H_{11}O_5Na;0,3H_2O$<br>MG=323,66 Fp:308-310 |
| 103 | 4'-$OCH_3$ | H | H | H | H | f. Säu: $S=C_{17}H_{12}O_5$<br>MG=296,28 Fp:187-190 |
| 104 | 3',4',5'-tri-$OCH_3$ | | | H | H | a) f. Säu: $S=C_{19}H_{16}O_7$<br>MG=356,33 Fp:260-262<br>b) Na-Salz: $S=C_{19}H_{15}O_7Na;1,8H_2O$<br>MG=410,74 Fp:206-210 |
| 105 | 3',4',5'-tri-OH | | | H | H | a) f. Säu: $S=C_{16}H_{10}O_7;1H_2O$<br>MG=332,27 am<br>b) Na-Salz: $S=C_{16}H_9O_7Na;1,1H_2O$<br>MG=356,05 am |
| 106 | 3',4'-di-OH | | H | H | H | f. Säu: $S=C_{16}H_{10}O_6$<br>MG=298,25 Fp:248-252 |
| 107 | 3'-Cl | H | H | 6-$CH_3$ | H | f. Säu: $S=C_{17}H_{11}O_4Cl$<br>MG=314,73 Fp:217-219 |
| 108 | 2'-Cl | H | H | H | H | f. Säu: $S=C_{16}H_9O_4Cl$<br>MG=300,70 Fp:140-143 |

| Bsp Nr. | $R^1$ | $R^2$ | $R^3$ | $R^4$ | $R^5$ | Bemerkungen<br>S=Summenformel<br>MG=Molekulargewicht<br>Fp. in °C |
|---|---|---|---|---|---|---|
| 109 | 4'-$OCH_3$ | H | H | 5,7-di-$OCH_3$ | | f. Säu: S=$C_{19}H_{16}O_7$<br>MG=356,35 Fp:234-238 |
| 110 | H | H | H | 5,7-di-$OCH_3$ | | f. Säu: S=$C_{18}H_{14}O_6$<br>MG=326,32 Fp:227-229 |
| 111 | 4'-OH | H | H | 5,7-di-OH | | f. Säu: S=$C_{16}H_{10}O_7$<br>MG=314,26 Fp:278-285 |
| 112 | 3',4'-di-$OCH_3$ | | H | H | H | f. Säu: S=$C_{18}H_{14}O_6$<br>MG=326,31 am |
| 113 | 4'-O-$(CH_2)_{15}$-$CH_3$ | H | H | H | H | f. Säu: $C_{32}H_{42}O_5$<br>MG=505,68 Fp:132-135 |
| 114 | 4'-$OCH_3$ | H | H | 7-$OCH_3$ | H | f. Säu: S=$C_{18}H_{14}O_6$<br>MG=326,31 Fp:239-245 |
| 115 | 4'-$OC_{20}H_{41}$ | H | H | H | H | f. Säu: S=$C_{36}H_{50}O_5$<br>MG=562,79 Fp:130-134 |

f. Säu: = freie Säure Na-Salz = Natriumsalz

Beispiel I:

3-{[4-(2-Pyridyl)-piperazin-1-yl]-carbonyl}-flavon-hydrochlorid enthaltende Tabletten.

Es werden Tabletten in folgender Zusammensetzung pro Tablette hergestellt:

| | |
|---|---|
| 3-{[4-(2-Pyridyl)-piperazin-1-yl]-carbonyl}-flavon-hydrochlorid | 20 mg |
| Maisstärke | 60 mg |
| Milchzucker | 135 mg |
| Gelatine (als 10%-ige Lösung) | 6 mg |

Der Wirkstoff, die Maisstärke und der Milchzucker werden mit der 10 %-igen Gelatine-Lösung eingedickt. Die Paste wird zerkleinert und das entstandene Granulat wird auf ein geeignetes Blech gebracht und getrocknet. Das getrocknete Granulat wird durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann zu Tabletten von 240 mg verpreßt.

## Patentansprüche

**1.** Flavon-3-carbonsäure-Verbindungen der allgemeinen Formel I

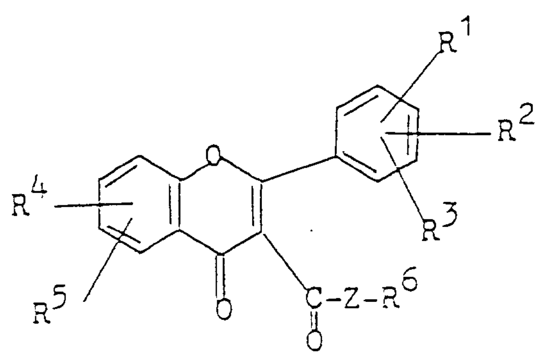

I

worin die Substituenten die folgenden Bedeutungen haben, wobei jedoch solche Substituentenkombinationen, welche aufgrund gegenseitiger sterischer Hinderung nicht möglich sind, ausgenommen sind, und

$R^1$ Wasserstoff, Alkyl mit 1-4 Kohlenstoffatomen, Halogen, Hydroxy, Alkylcarbonyloxy mit 2-5 Kohlenstoffatomen oder eine $R^9$-O-Gruppe, worin $R^9$ für eine Alkyl- oder Alkenylgruppe mit bis zu 20 Kohlenstoffatomen steht, bedeutet,

$R^2$ Wasserstoff, Alkyl mit 1-4 Kohlenstoffatomen, Halogen, Hydroxy, Alkylcarbonyloxy mit 2-5 Kohlenstoffatomen oder eine $R^9$-O-Gruppe, worin $R^9$ obige Bedeutung besitzt, bedeutet, und

$R^3$ Wasserstoff, Alkyl mit 1-4 Kohlenstoffatomen, Halogen, Hydroxy, Alkylcarbonyloxy mit 2-5 Kohlenstoffatomen oder eine $R^9$-O-Gruppe, worin $R^9$ obige Bedeutung besitzt, bedeutet, oder
von den Substituenten $R^1$ bis $R^3$ zwei an benachbarte Kohlenstoffatome gebunden sind und gemeinsam eine Alkylendioxygruppe mit 1-2 Kohlenstoffatomen bedeuten,
wobei jedoch, wenn mehrere der Substituenten $R^1$ bis $R^3$ sauerstoffhaltige Reste darstellen, diese Reste identisch sind,

$R^4$ Wasserstoff, Alkyl mit 1-4 Kohlenstoffatomen, Halogen, Hydroxy, Alkylcarbonyloxy mit

2-5 Kohlenstoffatomen oder eine $R^9$-O-Gruppe, worin $R^9$ obige Bedeutung besitzt, bedeutet, und

$R^5$ Wasserstoff, Alkyl mit 1-4 Kohlenstoffatomen, Halogen, Hydroxy, Alkylcarbonyloxy mit 2-5 Kohlenstoffatomen oder eine $R^9$-O-Gruppe, worin $R^9$ obige Bedeutung besitzt, bedeutet, oder

$R^4$ und $R^5$ an benachbarte Kohlenstoffatome gebunden sind und gemeinsam eine Alkylendioxygruppe mit 1-2 Kohlenstoffatomen bilden,

wobei jedoch, falls beide Substituenten $R^4$ und $R^5$ sauerstoffhaltige Reste darstellen, diese Reste identisch sind, und, falls $R^1$, $R^2$ und/oder $R^3$ Hydroxy oder Alkylcarbonyloxy-Gruppen mit 2-5 Kohlenstoffatomen darstellen, sauerstoffhaltige Reste $R^4$ und/oder $R^5$ mit diesen Gruppen identisch sind,

$R^6$ eine durch Alkyl mit 1-4 Kohlenstoffatomen disubstituierte Aminogruppe, eine Pyridyl- oder Pyrimidylgruppe, eine 1-Benzylpiperidin-4-yl-Gruppe oder die Gruppe a darstellt,

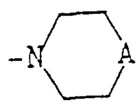

a

worin

A eine Bindung, die Methylengruppe, Sauerstoff oder eine N-$R^7$-Gruppe, bedeutet, worin

$R^7$ Wasserstoff, Alkyl mit 1-4 Kohlenstoffatomen, eine Pyridyl- oder Pyrimidylgruppe oder eine gegebenenfalls durch Alkyl mit 1-4 Kohlenstoffatomen oder Halogen substituierte Benzyl- oder Phenylgruppe bedeutet,

Z eine Y-$(CH_2)_n$-Gruppe bedeutet, worin

Y eine $NR^8$-Gruppe, worin $R^8$ Wasserstoff oder Alkyl mit 1-4 Kohlenstoffatomen ist, oder, falls keiner der Reste $R^1$-$R^5$ Hydroxy oder Alkylcarbonyloxy mit 2-5 Kohlenstoffatomen ist, Y auch Sauerstoff bedeutet, und

n eine ganze Zahl von 2-4 oder, falls $R^6$ eine 1-Benzylpiperidin-4-yl-Gruppe ist, auch Null bedeutet, oder

Z auch eine Bindung bedeutet, falls $R^6$ für eine Gruppe a steht, worin A eine N-$R^7$-Gruppe ist,

sowie N-Oxide und/oder Säureadditionssalze der Verbindungen der Formel I.

**2.** Flavon-3-carbonsäure-Verbindungen gemäß Anspruch 1, worin $R^1$ Alkoxy mit 1-4 Kohlenstoffatomen oder Hydroxy, $R^2$ Wasserstoff, Alkoxy mit 1-4 Kohlenstoffatomen oder Hydroxy und $R^3$ Wasserstoff, Alkoxy mit 1-4 Kohlenstoffatomen oder Hydroxy bedeuten und $R^4$ und $R^5$ Wasserstoff bedeuten.

**3.** Flavon-3-carbonsäure-Verbindungen gemäß Anspruch 2, worin $R^1$ und $R^2$ je Methoxy oder Hydroxy und $R^3$ Wasserstoff, Methoxy oder Hydroxy bedeuten.

**4.** Flavon-3-carbonsäure-Verbindungen gemäß Anspruch 1, 2 oder 3, worin $R^6$ eine Gruppe a) darstellt, worin A eine $NR^7$-Gruppe bedeutet, und Z für eine Bindung steht.

**5.** Flavon-3-carbonsäure-Verbindungen gemäß Anspruch 1, 2 oder 3, worin Z eine $Y(CH_2)_n$-Gruppe bedeutet, worin Y für NH steht und n für 2 - 4 steht.

**6.** Flavon-3-carbonsäure-Verbindungen gemäß Anspruch 5, worin n für 4 steht und $R^6$ einen 4-(Pyridyl)-piperazin-Rest bedeutet.

**7.** Arzneimittel enthaltend eine pharmakologisch wirksame Menge einer Flavon-3-carbonsäure-Verbindung gemäß Anspruch 1 und übliche pharmazeutische Hilfs- und/oder Trägerstoffe.

**8.** Verfahren zur Herstellung von Verbindungen der allgemeinen Formel I gemäß Anspruch 1

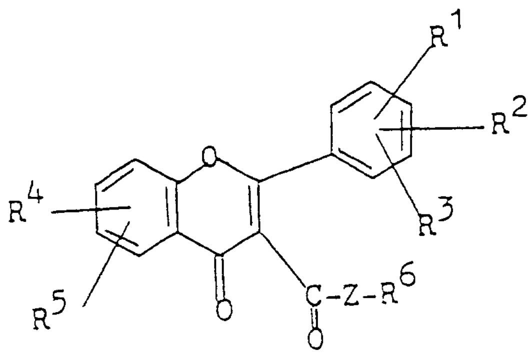

I

worin $R^1$, $R^2$, $R^3$, $R^4$, $R^5$, $R^6$ und Z die in Anspruch 1 angegebene Bedeutung besitzen, sowie deren N-Oxiden und/oder Säureadditionssalzen, dadurch gekennzeichnet, daß man

a) zur Herstellung von Verbindungen der allgemeinen Formel Ia

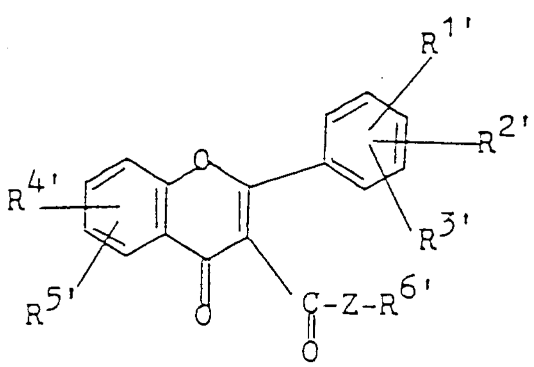

Ia

worin $R^{1'}$, $R^{2'}$, $R^{3'}$, $R^{4'}$ und $R^{5'}$ die für $R^1$, $R^2$, $R^3$, $R^4$ und $R^5$ angegebenen Bedeutungen mit Ausnahme von Alkylcarbonyloxy mit 2-5 Kohlenstoffatomen besitzen, Z obige Bedeutung besitzt und $R^{6'}$ die für $R^6$ angegebene Bedeutung mit Ausnahme solcher Gruppen, worin $R^7$ Wasserstoff bedeutet, besitzt, reaktive Derivate von Säuren der allgemeinen Formel II

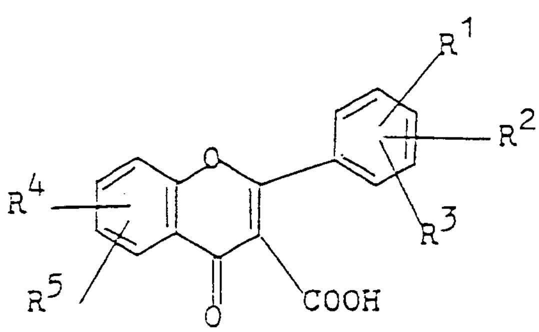

II

worin $R^1$, $R^2$, $R^3$, $R^4$ und $R^5$ obige Bedeutung besitzen, jedoch allfällige Hydroxygruppen mit einer Schutzgruppe versehen sind, mit Verbindungen der allgemeinen Formel III

$H\text{-}Z\text{-}R^{6'}$ III

worin $R^{6'}$ und Z obige Bedeutung besitzen, umsetzt, oder

b) zur Herstellung von Verbindungen der allgemeinen Formel Ib

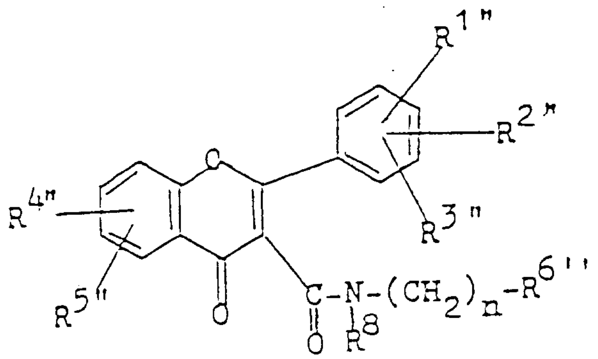

Ib

worin n und $R^8$ obige Bedeutung besitzen, $R^{1}$", $R^{2}$", $R^{3}$", $R^{4}$" und $R^{5}$" die für $R^1$, $R^2$, $R^3$, $R^4$ und $R^5$ angegebene Bedeutung mit Ausnahme von Hydroxy und Alkylcarbonyloxy mit 2-5 Kohlenstoffatomen besitzen, und $R^{6}$" eine durch Alkyl mit 1-4 Kohlenstoffatomen disubstituierte Aminogruppe oder die oben definierte Gruppe a bedeutet, Verbindungen der allgemeinen Formel IV

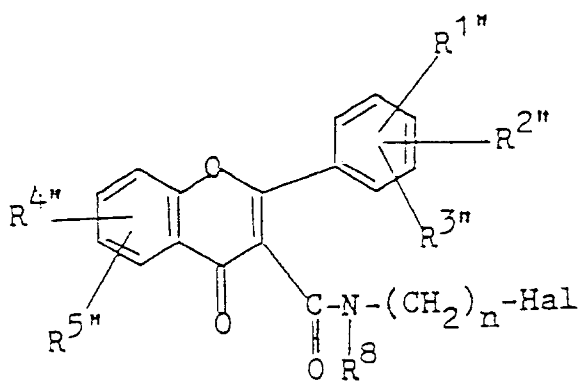

IV

worin $R^{1}$", $R^{2}$", $R^{3}$", $R^{4}$", $R^{5}$", n und $R^8$ obige Bedeutung besitzen und Hal Halogen bedeutet, mit Aminen der allgemeinen Formel V

H-$R^{6}$"    V

worin $R^{6}$" obige Bedeutung besitzt, umsetzt, und

gewünschtenfalls zur Herstellung von Verbindungen der Formel I, worin $R^1$, $R^2$, $R^3$, $R^4$ und/oder $R^5$ Alkylcarbonyloxy-Gruppen mit 2-5 Kohlenstoffatomen bedeuten, in Verbindungen der Formel Ia, worin $R^{1'}$, $R^{2'}$, $R^{3'}$, $R^{4'}$ und/oder $R^{5'}$ freie Hydroxygruppen bedeuten, diese zu Alkylcarbonyloxy-Gruppen mit 2-5 Kohlenstoffatomen acyliert und/oder gewünschtenfalls zur Herstellung von Verbindungen der Formel I, worin $R^1$, $R^2$, $R^3$, $R^4$ und/oder $R^5$ freie Hydroxygruppen bedeuten, in Verbindungen der Formel I, worin $R^1$, $R^2$, $R^3$, $R^4$ und/oder $R^5$ Alkoxygruppen mit 1-4 Kohlenstoffatomen bedeuten, diese in freie Hydroxygruppen überführt und/oder zur Herstellung von Verbindungen der Formel I, worin $R^7$ Wasserstoff bedeutet, aus erhaltenen Verbindungen der Formel I, worin $R^7$ Benzyl bedeutet, die Benzylgruppe abspaltet, und gewünschtenfalls Verbindungen der allgemeinen Formel I zu ihren N-Oxiden oxidiert und/oder in ihre Säureadditionssalze überführt oder die Säureadditionssalze in die freien Verbindungen überführt.

**9.** 3-Flavoncarbonsäuren der allgemeinen Formel II

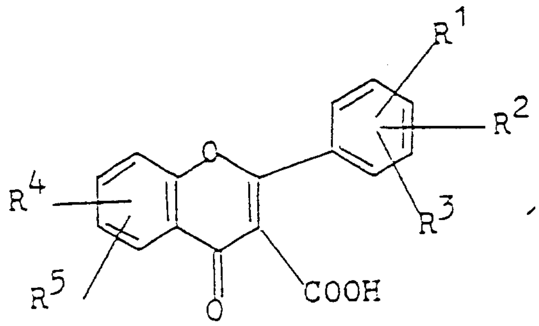

, II

worin die Substituenten die folgenden Bedeutungen haben, wobei jedoch solche Substituentenkombinationen, welche aufgrund gegenseitiger sterischer Hinderung nicht möglich sind, ausgenommen sind, und

$R^1$ Wasserstoff, Alkyl mit 1-4 Kohlenstoffatomen, Halogen, Hydroxy, Alkylcarbonyloxy mit 2-5 Kohlenstoffatomen oder eine $R^9$-O-Gruppe, worin $R^9$ für eine Alkyl- oder Alkenylgruppe mit bis zu 20 Kohlenstoffatomen steht, bedeutet,

$R^2$ Wasserstoff, Alkyl mit 1-4 Kohlenstoffatomen, Halogen, Hydroxy, Alkylcarbonyloxy mit 2-5 Kohlenstoffatomen oder eine $R^9$-O-Gruppe, worin $R^9$ obige Bedeutung besitzt, bedeutet, und

$R^3$ Wasserstoff, Alkyl mit 1-4 Kohlenstoffatomen, Halogen, Hydroxy, Alkylcarbonyloxy mit 2-5 Kohlenstoffatomen oder eine $R^9$-O-Gruppe, worin $R^9$ obige Bedeutung besitzt, bedeutet, wobei jedoch ein in 2'-Stellung stehender Substituent nur dann Methoxy bedeutet, wenn mindestens einer der übrigen Substituenten $R^1$-$R^5$, nicht Wasserstoff bedeutet, oder

von den Substituenten $R^1$ bis $R^3$ zwei an benachbarte Kohlenstoffatome gebunden sind und gemeinsam eine Alkylendioxygruppe mit 1-2 Kohlenstoffatomen bedeuten, wobei jedoch, wenn mehrere der Substituenten $R^1$ bis $R^3$ sauerstoffhaltige Reste darstellen, diese Reste identisch sind,

$R^4$ Wasserstoff, Alkyl mit 1-4 Kohlenstoffatomen, Halogen, Hydroxy, Alkylcarbonyloxy mit 2-5 Kohlenstoffatomen oder eine $R^9$-O-Gruppe, worin $R^9$ obige Bedeutung besitzt, bedeutet, und

$R^5$ Wasserstoff, Alkyl mit 1-4 Kohlenstoffatomen, Halogen, Hydroxy, Alkylcarbonyloxy mit 2-5 Kohlenstoffatomen oder eine $R^9$-O-Gruppe, worin $R^9$ obige Bedeutung besitzt, bedeutet, oder

$R^4$ und $R^5$ an benachbarte Kohlenstoffatome gebunden sind und gemeinsam eine Alkylenkette mit 1-2 Kohlenstoffatomen bilden,

wobei jedoch, falls $R^4$ und $R^5$ sauerstoffhaltige Reste darstellen, diese Reste identisch sind, und, falls $R^1$, $R^2$ und/oder $R^3$ Hydroxy darstellen, sauerstoffhaltige Reste $R^4$ und/oder $R^5$ ebenfalls Hydroxy darstellen, und wobei mindestens einer der Substituenten $R^1$ bis $R^5$ nicht Wasserstoff bedeutet, und, falls $R^1$, $R^2$ und $R^3$ Wasserstoff und $R^4$ 5-Methyl bedeuten, $R^5$ nicht 7-Methyl bedeutet, und deren Salze.

## Claims

1. Flavone-3-carboxylic acid compounds of the general Formula I,

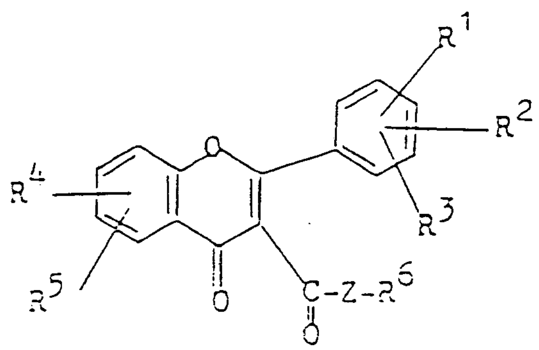

I

wherein the substituents have the following meanings, but those combinations of substituents which are not possible owing to mutual steric hindrance being excepted, and

| | |
|---|---|
| $R^1$ | represents hydrogen, alkyl with 1-4 carbon atoms, halogen, hydroxy, alkylcarbonyloxy with 2-5 carbon atoms or an $R^9$-O group, wherein $R^9$ stands for an alkyl or alkenyl group with up to 20 carbon atoms, |
| $R^2$ | represents hydrogen, alkyl with 1-4 carbon atoms, halogen, hydroxy, alkylcarbonyloxy with 2-5 carbon atoms or an $R^9$-O group, wherein $R^9$ has the above meaning, and |
| $R^3$ | represents hydrogen, alkyl with 1-4 carbon atoms, halogen, hydroxy, alkylcarbonyloxy with 2-5 carbon atoms or an $R^9$-O group, wherein $R^9$ has the above meaning, or of the substituents $R^1$ to $R^3$ two are bonded to adjacent carbon atoms and together represent an alkylenedioxy group with 1-2 carbon atoms, but if several of the substituents $R^1$ to $R^3$ represent oxygen-containing radicals, these radicals are identical, |
| $R^4$ | represents hydrogen, alkyl with 1-4 carbon atoms, halogen, hydroxy, alkylcarbonyloxy with 2-5 carbon atoms or an $R^9$-O group, wherein $R^9$ has the above meaning, and |
| $R^5$ | represents hydrogen, alkyl with 1-4 carbon atoms, halogen, hydroxy, alkylcarbonyloxy with 2-5 carbon atoms or an $R^9$-O group, wherein $R^9$ has the above meaning, or |
| $R^4$ and $R^5$ | are bonded to adjacent carbon atoms and together form an alkylenedioxy group with 1-2 carbon atoms, but if both substituents $R^4$ and $R^5$ represent oxygen-containing radicals, these radicals are identical, and, if $R^1$, $R^2$ and/or $R^3$ represent hydroxy or alkylcarbonyloxy groups with 2-5 carbon atoms, oxygen-containing radicals $R^4$ and/or $R^5$ are identical to these groups, |
| $R^6$ | represents an amino group disubstituted by alkyl with 1-4 carbon atoms, a pyridyl or pyrimidyl group, a 1-benzyl-piperidin-4-yl group or the group a, |

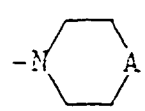

a

wherein

| | |
|---|---|
| A | represents a bond, the methylene group, oxygen or an N-$R^7$ group, wherein |
| $R^7$ | represents hydrogen, alkyl with 1-4 carbon atoms, a pyridyl or pyrimidyl group or a benzyl or phenyl group optionally substituted by alkyl with 1-4 carbon atoms or halogen, |
| Z | represents a Y-$(CH_2)_n$ group, wherein |
| Y | represents an $NR^8$ group, wherein $R^8$ is hydrogen or alkyl with 1-4 carbon atoms, or, if none of the radicals $R^1$-$R^5$ is hydroxy or alkylcarbonyloxy with 2-5 carbon atoms, Y also represents oxygen, and |

n represents a whole number from 2 to 4 or, if $R^6$ is a 1-benzylpiperidin-4-yl group, also represents zero, or

Z also represents a bond, if $R^6$ stands for a group a, wherein A is an N-$R^7$ group,

and N-oxides and/or acid addition salts of compounds of Formula I.

**2.** Flavone-3-carboxylic acid compounds according to Claim 1, wherein $R^1$ represents alkoxy with 1-4 carbon atoms or hydroxy, $R^2$ represents hydrogen, alkoxy with 1-4 carbon atoms or hydroxy and $R^3$ represents hydrogen, alkoxy with 1-4 carbon atoms or hydroxy and $R^4$ and $R^5$ represent hydrogen.

**3.** Flavone-3-carboxylic acid compounds according to Claim 2, wherein $R^1$ and $R^2$ each represent methoxy or hydroxy and $R^3$ represents hydrogen, methoxy or hydroxy.

**4.** Flavone-3-carboxylic acid compounds according to Claim 1, 2 or 3, wherein $R^6$ denotes a group (a), wherein A represents an $NR^7$ group and Z stands for a bond.

**5.** Flavone-3-carboxylic acid compounds according to Claim 1, 2 or 3, wherein Z represents a $Y(CH_2)_n$ group, wherein Y stands for NH and n stands for 2-4.

**6.** Flavone-3-carboxylic acid compounds according to Claim 5, wherein n stands for 4 and $R^6$ represents a 4-(pyridyl)-piperazine radical.

**7.** Medicament containing a pharmacologically effective amount of a flavone-3-carboxylic acid compound according to Claim 1 and conventional pharmaceutical auxiliaries and/or carriers.

**8.** Process for the preparation of compounds of the general Formula I in accordance with Claim 1,

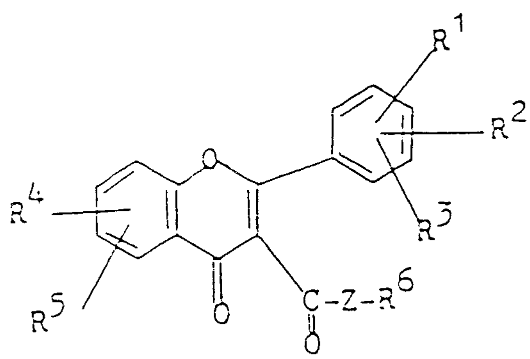

I

wherein $R^1$, $R^2$, $R^3$, $R^4$, $R^5$, $R^6$ and Z have the meanings given in Claim 1, and also the N-oxides and/or acid addition salts thereof, characterised in that

a) for the preparation of compounds of the general Formula Ia

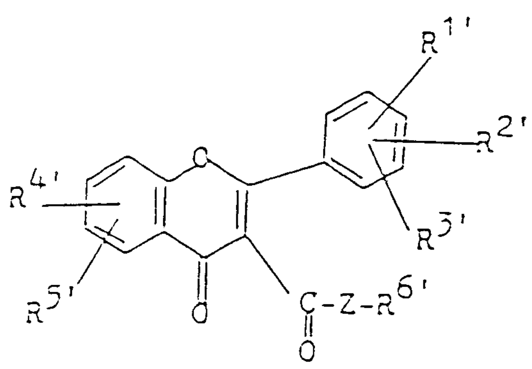

Ia

wherein $R^{1'}$, $R^{2'}$, $R^{3'}$, $R^{4'}$ and $R^{5'}$ have the meanings given for $R^1$, $R^2$, $R^3$, $R^4$ and $R^5$ with the

exception of alkylcarbonyloxy with 2-5 carbon atoms, Z has the above meaning and $R^{6}$' has the meaning given for $R^6$ with the exception of those groups wherein $R^7$ represents hydrogen, reactive derivatives of acids of general Formula II,

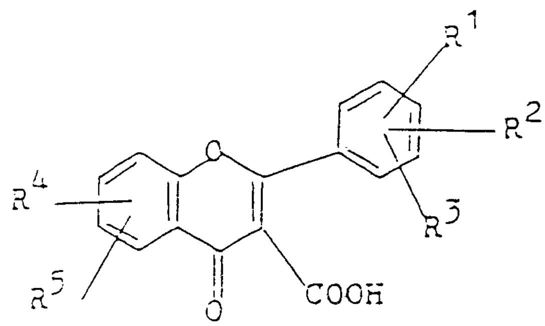

II

wherein $R^1$, $R^2$, $R^3$, $R^4$ and $R^5$ have the above meanings, but any hydroxy groups are provided with a protective group, are reacted with compounds of the general Formula III,

H-Z-$R^{6}$'　　III

wherein $R^{6}$' and Z have the above meanings, or
b) for the preparation of compounds of the general Formula Ib,

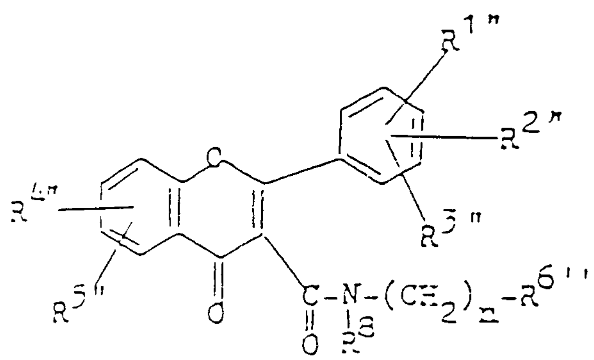

Ib

wherein n and $R^8$ have the above meanings, $R^{1}$", $R^{2}$", $R^{3}$", $R^{4}$" and $R^{5}$" have the meanings given for $R^1$, $R^2$, $R^3$, $R^4$ and $R^5$ with the exception of hydroxy and alkylcarbonyloxy with 2-5 carbon atoms, and $R^{6}$" represents an amino group disubstituted by alkyl with 1-4 carbon atoms or the above defined group a, compounds of the general Formula IV,

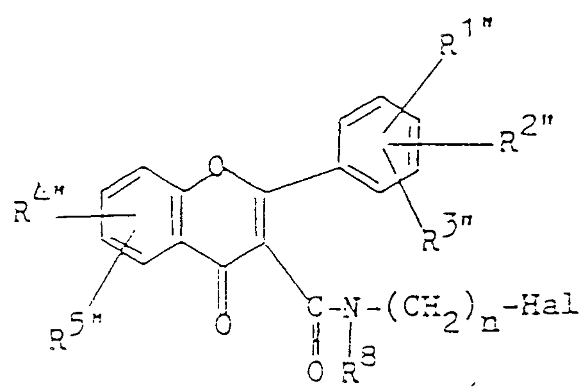

IV

wherein $R^{1''}$, $R^{2''}$, $R^{3''}$, $R^{4''}$, $R^{5''}$, n and $R^8$ have the above meanings and Hal represents halogen, are reacted with amines of the general Formula V,

$H\text{-}R^{6''}$ V

wherein $R^{6''}$ has the above meaning, and if desired for the preparation of compounds of Formula I wherein $R^1$, $R^2$, $R^3$, $R^4$ and/or $R^5$ represent alkylcarbonyloxy groups with 2-5 carbon atoms, in compounds of Formula Ia wherein $R^{1'}$, $R^{2'}$, $R^{3'}$, $R^{4'}$ and/or $R^{5'}$ represent free hydroxy groups, these are acylated to alkylcarbonyloxy groups with 2-5 carbon atoms and/or if desired for the preparation of compounds of Formula I wherein $R^1$, $R^2$, $R^3$, $R^4$ and/or $R^5$ represent free hydroxy groups, in compounds of Formula I wherein $R^1$, $R^2$, $R^3$, $R^4$ and/or $R^5$ represent alkoxy groups with 1-4 carbon atoms, these are converted into free hydroxy groups and/or for the preparation of compounds of Formula I wherein $R^7$ represents hydrogen the benzyl group is split off from the compounds of Formula I obtained wherein $R^7$ represents benzyl, and if desired, compounds of the general Formula I are oxidised to produce their N-oxides and/or converted into their acid addition salts or the acid addition salts are converted into the free compounds.

**9.** 3-Flavone carboxylic acids of the general Formula II,

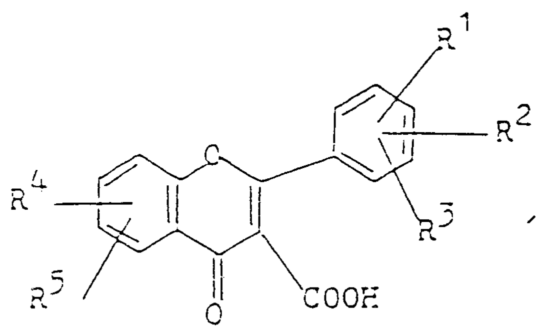

, II

wherein the substituents have the following meanings, but those combinations of substituents which are not possible owing to mutual steric hindrance being excepted, and

- $R^1$ represents hydrogen, alkyl with 1-4 carbon atoms, halogen, hydroxy, alkylcarbonyloxy with 2-5 carbon atoms or an $R^9$-O group, wherein $R^9$ stands for an alkyl or alkenyl group with up to 20 carbon atoms,
- $R^2$ represents hydrogen, alkyl with 1-4 carbon atoms, halogen, hydroxy, alkylcarbonyloxy with 2-5 carbon atoms or an $R^9$-O group, wherein $R^9$ has the above meaning, and
- $R^3$ represents hydrogen, alkyl with 1-4 carbon atoms, halogen, hydroxy, alkylcarbonyloxy with 2-5 carbon atoms or an $R^9$-O group, wherein $R^9$ has the above meaning, but a substituent present in the 2'-position only represents methoxy when at least one of the other substituents $R^1$ to $R^5$ does not represent hydrogen, or

of the substituents $R^1$ to $R^3$, two are bonded to adjacent carbon atoms and together represent an alkylenedioxy group with 1-2 carbon atoms, but if several of the substituents $R^1$ to $R^3$ represent oxygen-containing radicals, these radicals are identical,

- $R^4$ represents hydrogen, alkyl with 1-4 carbon atoms, halogen, hydroxy, alkylcarbonyloxy with 2-5 carbon atoms or an $R^9$-O group, wherein $R^9$ has the above meaning, and
- $R^5$ represents hydrogen, alkyl with 1-4 carbon atoms, halogen, hydroxy, alkylcarbonyloxy with 2-5 carbon atoms or an $R^9$-O group, wherein $R^9$ has the above meaning, or
- $R^4$ and $R^5$ are bonded to adjacent carbon atoms and together form an alkylene chain with 1-2 carbon atoms,

wherein, however, if $R^4$ and $R^5$ represent oxygen-containing radicals, these radicals are identical, and, if $R^1$, $R^2$ and/or $R^3$ represent hydroxy, oxygen-containing radicals $R^4$ and/or $R^5$ likewise represent hydroxy, and at least one of the substituents $R^1$ to $R^5$ does not represent hydrogen, and, if $R^1$, $R^2$ and $R^3$ represent hydrogen and $R^4$ represents 5-methyl, $R^5$ does not represent 7-methyl, and their salts.

## Revendications

1. Composés d'acide flavone-3-carboxylique de formule générale I

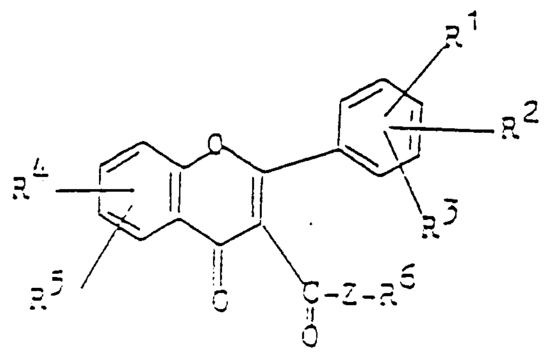

I

dans laquelle les substituants ont les significations suivantes, les combinaisons de substituants qui ne sont pas possibles en raison d'un empêchement stérique réciproque étant toutefois exclues,

$R^1$ signifie l'hydrogène, un alkyle avec 1 à 4 atomes de carbone, un halogène, un hydroxy, un alkylcarbonyloxy avec 2 à 5 atomes de carbone ou un groupe $R^9$-O-où $R^9$ représente un groupe alkyle ou alcényle avec jusqu'à 20 atomes de carbone,

$R^2$ signifie l'hydrogène, un alkyle avec 1 à 4 atomes de carbone, un halogène, un hydroxy, un alkylcarbonyloxy avec 2 à 5 atomes de carbone ou un groupe $R^9$-O- où $R^9$ possède la signification précédente,

$R^3$ signifie l'hydrogène, un alkyle avec 1 à 4 atomes de carbone, un halogène, un hydroxy, un alkylcarbonyloxy avec 2 à 5 atomes de carbone ou un groupe $R^9$-O- où $R^9$ a la signification précédente ou,

parmi les substituants $R^1$ à $R^3$, deux sont liés à des atomes de carbone voisins et représentent ensemble un groupe alkylènedioxy avec 1 a 2 atomes de carbone, moyennant quoi toutefois, lorsque plusieurs des substituants $R^1$ à $R^3$ représentent des restes contenant de l'oxygène, ces restes sont identiques,

$R^4$ signifie l'hydrogène, un alkyle avec 1 à 4 atomes de carbone, un halogène, un hydroxy, un alkylcarbonyloxy avec 2 à 5 atomes de carbone ou un groupe $R^9$-O- où $R^9$ a la signification précédente, et

$R^5$ signifie l'hydrogène, un alkyle avec 1 à 4 atomes de carbone, un halogène, un hydroxy, un alkylcarbonyloxy avec 2 à 5 atomes de carbone ou un groupe $R^9$-O- où $R^9$ a la signification précédente, ou

$R^4$ et $R^5$ sont liés à des atomes de carbone voisins et forment ensemble un groupe alkylènedioxy avec 1 à 2 atomes de carbone,

moyennant quoi toutefois, si les deux substituants $R^4$ et $R^5$ représentent des restes contenant de l'oxygène, ces restes sont identiques et, si $R^1$, $R^2$ et/ou $R^3$ représentent des groupes hydroxy ou alkylcarbonyloxy avec 2 à 5 atomes de carbone, les restes $R^4$ et/ou $R^5$ contenant de l'oxygène sont identiques à ces groupes,

$R^6$ représente un groupe amino disubstitué par un alkyle avec 1 à 4 atomes de carbone, un groupe pyridyle ou pyrimidyle, un groupe 1-benzylpipéridine-4-yle ou le groupe a

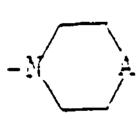

a

dans lequel

A signifie une liaison, le groupe méthylène, l'oxygène ou un groupe N-$R^7$ où

$R^7$ signifie l'hydrogène, un alkyle avec 1 à 4 atomes de carbone, un groupe pyridyle ou pyrimidyle ou un groupe benzyle ou phényle éventuellement substitué par un alkyle ayant 1 à 4 atomes de

carbone ou par un halogène,

Z signifie un groupe Y-$(CH_2)_n$- où Y signifie un groupe $NR^8$ où $R^8$ est l'hydrogène ou un alkyle avec 1 à 4 atomes de carbone ou, si aucun des restes $R^1$-$R^5$ n'est un hydroxy ou un alkylcarbonyloxy avec 2 à 5 atomes de carbone, Y signifie également de l'oxygène et

n représente un nombre entier de 2 à 4 ou, si $R^6$ est un groupe 1-benzylpipéridine-4-yle, est également égal à zéro, ou

Z représente également une liaison si $R^6$ représente un groupe a où A est un groupe N-$R^7$, ainsi que des N-oxydes et/ou des sels d'addition d'acides des composés de formule I.

**2.** Composés d'acide flavone-3-carboxylique selon la revendication 1, dans lesquels $R^1$ signifie un alcoxy avec 1 à 4 atomes de carbone ou un hydroxy, $R^2$ signifie l'hydrogène, un alcoxy avec 1 à 4 atomes de carbone ou un hydroxy, et $R^3$ signifie l'hydrogène, un alcoxy avec 1 à 4 atomes de carbone ou un hydroxy, et $R^4$ et $R^5$ signifient l'hydrogène.

**3.** Composés d'acide flavone-3-carboxylique selon la revendication 2, dans lesquels $R^1$ et $R^2$ signifient chacun un méthoxy ou un hydroxy et $R^3$ signifie l'hydrogène, un méthoxy ou un hydroxy.

**4.** Composés d'acide flavone-3-carboxylique selon la revendication 1, 2 ou 3, dans lesquels $R^6$ représente un groupe a) où A signifie un groupe $NR^7$ et Z représente une liaison.

**5.** Composés d'acide flavone-3-carboxylique selon la revendication 1, 2 ou 3, dans lesquels Z signifie un groupe $Y(CH_2)_n$ où Y représente NH et n est égal à 2-4.

**6.** Composés d'acide flavone-3-carboxylique selon la revendication 5, dans lesquels n est égal à 4 et $R^6$ signifie un reste 4-(pyridyl)-pipérazine.

**7.** Médicament contenant une quantité pharmaceutiquement efficace d'un composé d'acide flavone-3-carboxylique selon la revendication 1 et les adjuvants et/ou excipients pharmaceutiques usuels.

**8.** Procédé de préparation de composés de formule générale I selon la revendication 1

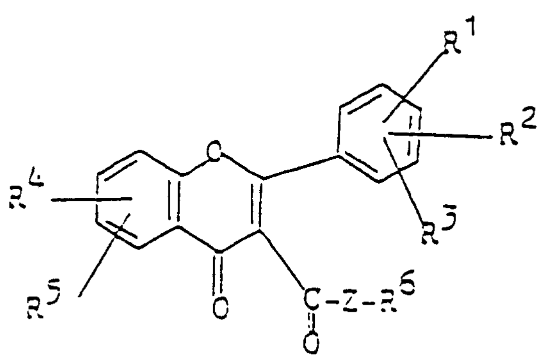

I

dans laquelle $R^1$, $R^2$, $R^3$, $R^4$, $R^5$, $R^6$ et Z ont la signification indiquée dans la revendication 1, ainsi que leurs N-oxydes et/ou sels d'addition d'acides, caractérisé en ce que,

a) pour la préparation de composés de formule générale Ia

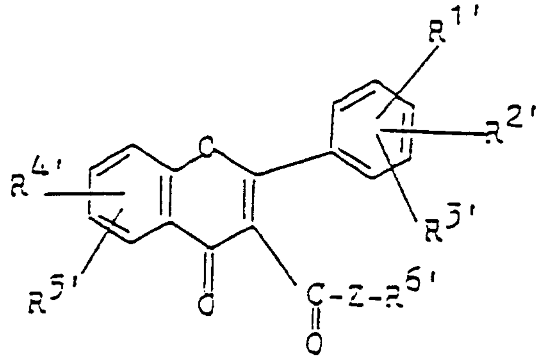

Ia

dans laquelle $R^{1'}$, $R^{2'}$, $R^{3'}$, $R^{4'}$ et $R^{5'}$ ont les significations indiquées à propos de $R^1$, $R^2$, $R^3$, $R^4$ et $R^5$, à l'exception d'un alkylcarbonyloxy avec 2 à 5 atomes de carbone, Z possède la signification précédente et $R^{6'}$ possède la signification indiquée à propos de $R^6$, à l'exception de groupes où $R^7$ signifie l'hydrogène, on fait réagir des dérivés réactifs d'acides de formule générale II

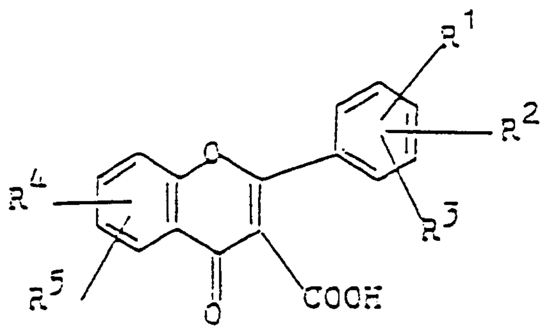

II

dans laquelle $R^1$, $R^2$, $R^3$, $R^4$ et $R^5$ ont la signification précédente, les groupes hydroxyles éventuels étant cependant pourvus d'un groupe protecteur, avec des composés de formule générale III

$H\text{-}Z\text{-}R^{6'}$ III

dans laquelle $R^{6'}$ et Z ont la signification précédente ou que,
b) pour la préparation de composés de formule générale Ib

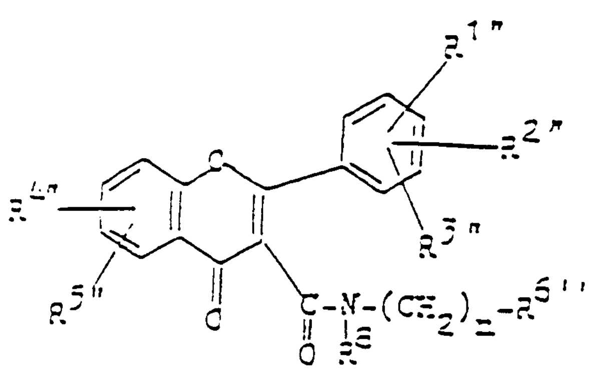

Ib

dans laquelle n et $R^8$ ont la signification précédente, $R^{1''}$, $R^{2''}$, $R^{3''}$, $R^{4''}$ et $R^{5''}$ ont la signification indiquée à propos de $R^1$, $R^2$, $R^3$, $R^4$ et $R^5$, à l'exception d'un hydroxy et d'un alkylcarbonyloxy avec 2 à 5 atomes de carbone et $R^{6''}$ signifie un groupe amino disubstitué par un alkyle ayant 1 à 4

atomes de carbone ou le groupe a défini plus haut, on fait réagir des composés de formule générale IV

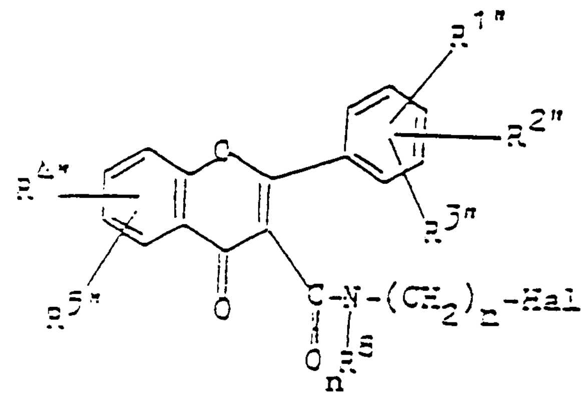

IV

dans laquelle $R^{1}$", $R^{2}$", $R^{3}$", $R^{4}$", $R^{5}$", n et $R^{8}$ possèdent la signification précédente et Hal signifie un halogène, avec des amines de formule générale V

H-$R^{6}$"  V

dans laquelle $R^{6}$" a la signification précédente et que, le cas échéant, pour la préparation de composés de formule I où $R^{1}$, $R^{2}$, $R^{3}$ $R^{4}$, et/ou $R^{5}$ signifient des groupes alkylcarbonyloxy avec 2 à 5 atomes de carbone, en composés de formule Ia où $R^{1}$', $R^{2}$', $R^{3}$', $R^{4}$' et/ou $R^{5}$' représentent des groupes hydroxyles libres, on acyle ceux-ci en groupes alkylcarbonyloxy comportant 2 à 5 atomes de carbone et/ou, le cas échéant, pour la préparation de composés de formule I où $R^{1}$, $R^{2}$, $R^{3}$, $R^{4}$ et/ou $R^{5}$ signifient des groupes hydroxyles libres, en composés de formule I dans laquelle $R^{1}$, $R^{2}$, $R^{3}$, $R^{4}$ et/ou $R^{5}$ signifient des groupes alcoxy avec 1 à 4 atomes de carbone, on transforme ceux-ci en groupes hydroxyles libres et/ou pour la préparation de composés de formule I dans laquelle $R^{7}$ signifie l'hydrogène, on élimine le groupe benzyle des composés de formule I obtenus où $R^{7}$ signifie un benzyle et, le cas échéant, on oxyde les composés de formule générale I en leurs N-oxydes et/ou les convertit en leurs sels d'addition d'acides ou transforme les sels d'addition d'acides en composés libres.

**9.** Acides 3-flavone carboxyliques de formule générale II

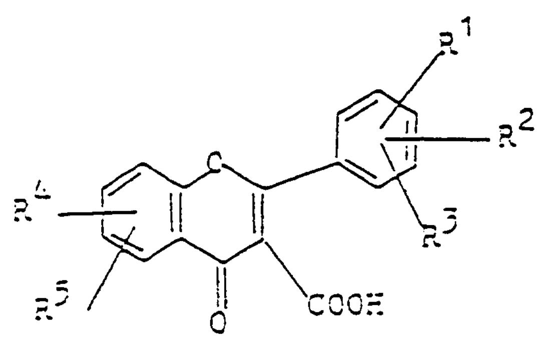

II

dans laquelle les substituants ont les significations suivantes, les combinaisons de substituants qui ne sont pas possibles en raison d'un empêchement stérique réciproque étant toutefois exclues, et

$R^{1}$ signifie l'hydrogène, un alkyle avec 1 à 4 atomes de carbone, un halogène, un hydroxy, un alkylcarbonyloxy avec 2 à 5 atomes de carbone ou un groupe $R^{9}$-O- où $R^{9}$ représente un groupe alkyle ou alcényle avec jusqu'à 20 atomes de carbone,

$R^{2}$ signifie l'hydrogène, un alkyle avec 1 à 4 atomes de carbone, un halogène, un hydroxy, un alkylcarbonyloxy avec 2 à 5 atomes de carbone ou un groupe $R^{9}$-O- où $R^{9}$ possède la signification précédente,

$R^3$ signifie l'hydrogène, un alkyle avec 1 à 4 atomes de carbone, un halogène, un hydroxy, un alkylcarbonyloxy avec 2 à 5 atomes de carbone ou un groupe $R^9$-O- où $R^9$ a la signification précédente, auquel cas toutefois un substituant se trouvant en position 2' ne représente alors un méthoxy que lorsqu'au moins un des substituants restants $R^1$-$R^5$ ne signifie pas l'hydrogène ou, parmi les substituants $R^1$ à $R^3$ deux sont liés à des atomes de carbone voisins et représentent ensemble un groupe alkylènedioxy avec 1 à 2 atomes de carbone, moyennant quoi toutefois, lorsque plusieurs des substituants $R^1$ à $R^3$ représentent des restes contenant de l'oxygène, ces restes sont identiques,

$R^4$ signifie l'hydrogène, un alkyle avec 1 à 4 atomes de carbone, un halogène, un hydroxy, un alkylcarbonyloxy avec 2 à 5 atomes de carbone ou un groupe $R^9$-O- où $R^9$ a la signification précédente, et

$R^5$ signifie l'hydrogène, un alkyle avec 1 à 4 atomes de carbone, un halogène, un hydroxy, un alkylcarbonyloxy avec 2 à 5 atomes de carbone ou un groupe $R^9$-O- où $R^9$ a la signification précédente, ou

$R^4$ et $R^5$ sont liés à des atomes de carbone voisins et forment ensemble une chaîne alkylène avec 1 à 2 atomes de carbone, moyennant quoi toutefois, si $R^4$ et $R^5$ représentent des restes contenant de l'oxygène, ces restes sont identiques et, si $R^1$, $R^2$ et/ou $R^3$ représentent des hydroxy, les restes $R^4$ et/ou $R^5$ contenant de l'oxygène représentent également des hydroxy, auquel cas au moins un des substituants $R^1$ à $R^5$ ne signifie pas l'hydrogène et, si $R^1$, $R^2$ et $R^3$ signifient l'hydrogène et $R^4$ signifie un 5-méthyle, $R^5$ ne représente pas un 7-méthyle, et leurs sels.